# EUROPEAN PATENT SPECIFICATION

(11) **EP 2 660 250 B1**
(45) Date of publication and mention of the grant of the patent: **14.11.2018**
(21) Application number: 12175113.5
(22) Date of filing: 05.07.2012
(51) Int. Cl.: C07K 14/47, C07K 14/705, A01K 67/027

(54) **TCR TRANSGENIC MOUSE MODEL FOR IMMUNE DISEASE**
TCR-TRANSGENES MAUSMODELL FÜR IMMUNKRANKHEITEN
MODÈLE DE SOURIS TRANSGÉNIQUE TCR DESTINÉ À UNE MALADIE IMMUNITAIRE

(30) Priority: 02.05.2012 EP 12166416
(43) Date of publication of application: 06.11.2013
(73) Proprietor: Deutsches Rheuma-Forschungszentrum Berlin, 10117 Berlin (DE)
(72) Inventor: Fillatreau, Simon, 10115 Berlin (DE); Uckert, Wolfgang, 13125 Berlin (DE); Kieback, Elisa, 10405 Berlin (DE); Anderton, Stephen, Edinburgh, EH105SP (GB); Lampropoulou, Vicky, 13355 Berlin (DE); Stervbo, Ulrik, 13353 Berlin (DE); Bunse, Mario, 10961 Berlin (DE)
(74) Representative: Hertin und Partner Rechts- und Patentanwälte PartG mbB

(56) References cited:
- WO-A1-99/16867
- MENDEL ITZHAK ET AL: "A novel protective model against experimental allergic encephalomyelitis in mice expressing a transgenic TCR-specific for myelin oligodendrocyte glycoprotein", JOURNAL OF NEUROIMMUNOLOGY, vol. 149, no. 1-2, April 2004 (2004-04), pages 10-21, XP002688425, ISSN: 0165-5728
- CARRIER YIJUN ET AL: "Th3 cells in peripheral tolerance. I. Induction of Foxp3-positive regulatory T cells by Th3 cells derived from TGF-beta T cell-transgenic mice", JOURNAL OF IMMUNOLOGY, vol. 178, no. 1, January 2007 (2007-01), pages 179-185, XP002688426, ISSN: 0022-1767
- POELLINGER BERNADETTE ET AL: "Spontaneous relapsing-remitting EAE in the SJL/J mouse: MOG-reactive transgenic T cells recruit endogenous MOG-specific B cells", JOURNAL OF EXPERIMENTAL MEDICINE, vol. 206, no. 6, June 2009 (2009-06), XP002688427, ISSN: 0022-1007, DOI: 10.1084/jem.20090299
- BETTELLI E ET AL: "Myelin Oligodendrocyte Glycoprotein-specific T Cell Receptor Transgenic Mice Develop Spontaneous Autoimmune Optic Neuritis", THE JOURNAL OF EXPERIMENTAL MEDICINE, ROCKEFELLER UNIVERSITY PRESS, US, vol. 197, no. 9, 5 May 2003 (2003-05-05), pages 1073-1081, XP003008390, ISSN: 0022-1007, DOI: 10.1084/JEM.20021603
- ANDERSON ANA C ET AL: "A transgenic model of central nervous system autoimmunity mediated by CD4+ and CD8+ T and B cells.", JOURNAL OF IMMUNOLOGY (BALTIMORE, MD. : 1950) 1 MAR 2012, vol. 188, no. 5, 1 March 2012 (2012-03-01) , pages 2084-2092, XP002688428, ISSN: 1550-6606
- Stervbo U, et al.: "COMPARISON OF THE ANTIGEN-RECOGNITION PROPERTIES OF MOG-REACTIVE PATHOGENIC AND PROTECTIVE CD4+ TCELLS", Eur. J. Immunol. 2009: Wednesday, Workshops European Journal of Immunology, 19 August 2009 (2009-08-19), pages S580-S582, XP002688429, Retrieved from the Internet: URL:http://onlinelibrary.wiley.com/doi/10. 1002/eji.200990254/pdf [retrieved on 2012-12-01]
- BETTELLI ESTELLE: "Building different mouse models for human MS.", ANNALS OF THE NEW YORK ACADEMY OF SCIENCES APR 2007, vol. 1103, April 2007 (2007-04), pages 11-18, XP002688430, ISSN: 0077-8923
- KOUSKOFF V ET AL: "CASSETTE VECTORS DIRECTING EXPRESSION OF T CELL RECPETOR GENES IN TRANSGENIC MICE", JOURNAL OF IMMUNOLOGICAL METHODS, ELSEVIER SCIENCE PUBLISHERS B.V.,AMSTERDAM, NL, vol. 180, no. 2, 27 March 1995 (1995-03-27), pages 273-280, XP000619156, ISSN: 0022-1759, DOI: 10.1016/0022-1759(95)00002-R
- MIX E ET AL: "Animal models of multiple sclerosis-Potentials and limitations", PROGRESS IN NEUROBIOLOGY, PERGAMON PRESS, GB, vol. 92, no. 3, 1 November 2010 (2010-11-01), pages 386-404, XP027504247, ISSN: 0301-0082, DOI: 10.1016/J.PNEUROBIO.2010.06.005 [retrieved on 2010-06-15]
- BETTINA SCHREINER ET AL: "Modeling multiple sclerosis in laboratory animals", SEMINARS IN IMMUNOPATHOLOGY, SPRINGER, BERLIN, DE, vol. 31, no. 4, 3 October 2009 (2009-10-03), pages 479-495, XP019779253, ISSN: 1863-2300, DOI: 10.1007/S00281-009-0181-4
- TANJA SCHEIKL ET AL: "Transgenic mouse models of multiple sclerosis", CMLS CELLULAR AND MOLECULAR LIFE SCIENCES, BIRKHÄUSER-VERLAG, BA, vol. 67, no. 23, 17 August 2010 (2010-08-17), pages 4011-4034, XP019862282, ISSN: 1420-9071, DOI: 10.1007/S00018-010-0481-9
- DATABASE Geneseq [Online] 19 July 1999 (1999-07-19), "Mouse A1 T cell receptor alpha chain.", retrieved from EBI accession no. GSP:AAY05727 Database accession no. AAY05727

## Description

The invention relates to the field of transgenic mice, methods for investigating T-cell therapeutic function and screening active substances useful in the treatment of immune diseases, preferably autoimmune diseases. The invention relates to a transgenic mouse comprising a transgenic expression construct coding for a MOG-binding T-cell receptor (TCR) β chain, a T-cell receptor (TCR) protein that encodes said TCR that is isolated from said transgenic mouse and a method for screening one or more factors for involvement in T-cell therapeutic function in an immune disease.

### BACKGROUND

The immune system provides crucial defences against infections, but it can also cause severe diseases when reacting against self-tissues. All healthy individuals carry mature self-reactive lymphocytes in the periphery, and these cells have the potential to provoke disease. However, most individuals will not develop autoimmune disease because their autoreactive lymphocytes are constantly suppressed by peripheral tolerance mechanisms, among which natural regulatory (Treg) cells have an essential role. Multiple studies have documented defects in the suppressive function of Treg cells in patients with autoimmune diseases, suggesting that impaired Treg function might contribute to development or progression of autoimmune diseases. Conversely, there is clear evidence that conventional T cells are often pathogenic players during autoimmune diseases. It is therefore highly attractive to develop novel therapies aiming at increasing the protective function of Treg cells, or decreasing the pathogenic activity of Tconv cells in autoimmune diseases.

Treg cells present with interesting features for clinical application. Once activated, Treg cells can locally suppress CD4⁺ T cells specific for different antigens through dominant mechanisms of tolerance. In addition, they can also suppress CD8⁺ T cells, B cells, and innate immune cells, thereby targeting multiple aspects of immunopathogenesis. Proof of concept studies have demonstrated that adoptive Treg cell transfer could treat autoimmune disease in pre-clinical disease models. Importantly, two recent clinical trials demonstrated the possibility of limiting immune-mediated disease through administration of low-dose IL-2, which resulted in a marked increase in the frequency of Treg cells in treated individuals. This approach is however limited by the fact that IL-2 is not a selective Treg activation agent, as it can also target conventional T cells or other components of the immune system such as NK cells. As a result, only low doses of IL-2 can be administered to patients, which constrain the possibilities of increasing the efficacy of this therapy. It is therefore of great interest to develop novel approaches to harness the suppressive function of Treg cells for the treatment of autoimmune disorders, or other immune-mediated diseases.

Elimination of pathogenic T cells is another attractive strategy for treating autoimmune diseases. Drugs depleting conventional T cells (and other immune cells) such as Campath-1H, or preventing their accumulation in target organs such as Natalizumab, can limit disease progression in patients with autoimmune diseases. However, these broad-based therapies can lead to impairment of immunity against pathogens, and have severe side effects. It is therefore crucial to develop novel strategy to target the T cells driving autoimmune pathogenesis, without affecting the activated T cells providing protection from infectious diseases, including opportunistic pathogens.

The prior art discloses transgenic mice that express a T cell receptor (TCR) that binds specifically to a peptide of the myelin oligodendrocyte glycoprotein (MOG) (as described in Mendel, Itzahk et al., Journal of Neuroimmunology, 2004, 149, 1-2; Carrier, Yijun et al., Journal of Immunology, 2007, 178, 1; Poellinger, Bernadette et al., Journal of Experimental Medicine, 2009, 206, 6; Bettelli E. et al., The Journal of Experimental Medicine, 2003, 197, 9; Anderson, Ana et al., Journal of Immunology, 2012, 188, 5) and various approaches towards constructing mouse models for human MS are known in the art (as described in Stervbo U. et al., European Journal of Immunology, 2009; Bettelli Estelle, Annals of the New York Academy of Sciences, 2007, 1103; Kouskoff V. et al., Journal of Immunological Methods, 1995, 180, 2; Mix E. et al., Progress in Neurobiology, 2010, 92, 3; Schreiner, Bettina et al., Seminars in Immunopathology, 2009, 31, 4; Scheikl, Tanja et al., CMLS Cellular and Molecular Life Sciences, 2010, 67, 23).

The development of novel therapeutic strategies to increase the protective function of Treg cells, or decrease the pathogenic activities of Tconv cells has so far been limited due to a lack of appropriate pre-clinical model for rapidly investigating how various genes and signalling pathways determined the protective and pathogenic functions of T cells in autoimmune diseases. Here, we report the development of novel tools that allow the rapid investigation of the mechanisms controlling the protective and pathogenic functions of Treg and Tconv cells, respectively, in autoimmune diseases.

### SUMMARY OF THE INVENTION

A novel TCR transgenic mouse has been developed (abbreviated as Kaa mouse) for investigating the pathogenic function of Tconv cells and the protective roles of Treg cells in experimental autoimmune encephalomyelitis (EAE), which is the primary pre-clinical model for relapsing-remitting multiple sclerosis (RR-MS).

T-cell function is crucial in regulating immune disorders, especially autoimmune disease, and the application of novel medicaments or therapies requires an assessment of their effect on T-cell function, especially with regard to either enhancing Treg function or repressing Tconv function, both of which can be seen as either important effects or goals to be achieved by novel therapeutic approaches. The present invention therefore provides a set of related tools for interrogating Treg and Tconv function, which are linked by a single inventive concept.

The invention enables a quantitative read-out of T-cell therapeutic function or T-cell pathogenicity during adoptive transfer experiments. Until now there had existed no appropriate model for assaying therapeutic T-cell effect, especially with regards to a quantitative measurement of T-cell effect on the disease model EAE. It was entirely unexpected that Treg cells isolated from the transgenic mouse, or Treg cells transfected with TCR isolated from the transgenic mouse, would show protective T-cell function. It was also surprising that Tconv cells isolated from the transgenic mouse, or Tconv cells transfected with TCR isolated from the transgenic mouse, would show pathogenic function to the degree observed in the experiments provide herein. This protective (for Treg) or pathogenic (for Tconv) effect by the modified T-cells of the present invention allows a quantitative assessment of T-cell function in adoptive transfer experiments.

The T-cell receptor (TCR) itself (both protein and nucleic acid that encodes for said receptor) isolated from the transgenic model represents a useful tool for investigating T-cell function, as is described in more detail below. The creation of a transgenic mouse allows isolation of the transgenic TCR with improved ease in comparison to isolation of these molecules from a wild type mouse. Importantly, the transgenic mouse has enabled identification of the associated TCR α chain sequences, which were previously unknown. The approach described herein is therefore a significant improvement on those tools known in the art. The identification and subsequent use of the MOG-binding TCR (TCR α and β chain sequences) has enabled creation of the specific mouse disease model as described herein, allowing quantitative measurement of T-cell function during adoptive transfer.

In light of the prior art the technical problem underlying the invention was the provision of means for investigating T-cell therapeutic function that overcomes the disadvantages of the prior art. This problem is solved by the features of the independent claims. Preferred embodiments of the present invention are provided by the dependent claims.

Therefore, an object of the invention is to provide a transgenic mouse comprising a transgenic expression construct that encodes a MOG-binding T-cell receptor (TCR) beta chain under control of a T-cell specific transcriptional promoter,
characterised in that the nucleotide sequence that encodes said MOG-binding TCR beta chain comprises a sequence that encodes a CDR3 amino acid sequence of SEQ ID No. 1, or an amino acid sequence variant of at least 80% sequence identity, preferably at least 90% sequence identity, to SEQ ID No. 1, whereby the variant exhibits MOG-binding.

According to the invention the transgenic mouse of the invention is characterised in that the nucleotide sequence that encodes said MOG-binding TCR β chain comprises a Vβ8.2-Jβ2.1 rearrangement, which encodes a CDR3 amino acid sequence of SEQ ID No. 1, or an amino acid sequence variant of at least 80% sequence identity, preferably at least 90% sequence identity, to SEQ ID No. 1, whereby the variant exhibits MOG-binding.

In a preferred embodiment the transgenic mouse of the invention is characterised in that the Vβ8.2-Jβ2.1 rearrangement comprises a nucleotide sequence that encodes an amino acid sequence of SEQ ID No. 2, or an amino acid sequence variant of at least 80% sequence identity, preferably at least 90% sequence identity, to SEQ ID No. 2, whereby the variant exhibits MOG-binding.

Also disclosed herein is a nucleic acid molecule isolated from a transgenic mouse as described herein encoding a MOG-binding TCR β chain and a TCR α chain sequence. Also disclosed herein is a nucleic acid molecule isolated from a transgenic mouse as described herein encoding a MOG-binding TCR β chain or a MOG-binding TCR α chain sequence.

A further aspect of the invention relates to the T-cell receptor (TCR) isolated from the transgenic mouse as described herein, preferably encoded by a nucleic acid molecule isolated from said mouse.

Disclosed herein is a T-cell receptor (TCR) comprising a TCR α chain sequence
- with a CD3 region sequence selected from Table A, or an amino acid sequence variant of at least 80% sequence identity, preferably at least 90% sequence identity, to anyone of the CD3 region sequences of Table A, whereby the variant exhibits MOG-binding, or
- with a VJ region sequence selected from Table A, or an amino acid sequence variant of at least 80% sequence identity, preferably at least 90% sequence identity, to any one of the VJ region sequences of Table A, whereby the variant exhibits MOG-binding.

In the present application the terms "alpha" and "α" are used interchangeably, as are the terms "beta" and "β".

The creation of a transgenic mouse expressing the TCR β chain described above enables isolation of a TCR or TCR-encoding nucleic acid from the mouse that is a novel product compared to those TCR described in the art. The TCR comprises of both TCR β and TCR α chains. By determining the sequence of the TCR β chain via the transgene, the corresponding α chain also exhibits MOG-binding. However, the specific sequence of the alpha chain is not a limiting feature of the invention. The TCR alpha chain can be any sequence isolated from the transgenic mouse that exhibits the desired function. It is required that the alpha chain exhibits functional interplay with the beta chain to exhibit MOG-binding. Therefore, the definition of the TCR by its isolation from the transgenic mouse defines a novel molecule with surprising properties, namely the protective (or pathogenic) effect in the mouse disease model EAE. Importantly, the alpha sequence is isolated from the transgenic mouse, which provides its defining functional characteristics, regardless of the specific alpha chain sequence. The specific sequences of the alpha chain are therefore preferred embodiments of the TCR of the present invention.

The TCR α chain sequences isolated from the transgenic mouse can be obtained via various methods known to those skilled in the art, for example by amplification strategies, using preferably PCR with primers of specific sequences that recognise and amplify the relevant alpha nucleic acid sequences.

A further aspect of the invention relates to a mouse cell derived or isolated from a mouse as described herein.

A further aspect relates to a cell as described above, characterised in that the cell is
- a regulatory T-cell (Treg), characterised by the markers CD4+, CD25+, and preferably Fox3p+, and optionally additionally characterised by expression of markers IL-10, TGF-β and/or IL-35, or
- a pathogenic T-cell (Tconv), characterised by the markers CD4+, CD25-, and preferably Fox3p-, and optionally additionally characterised by expression of markers IFN-gamma, GM-CSF, IL-17, IL-21, IL-22, TNF, IL-6, lymphotoxin, granzyme, and/or perforin.

A further aspect of the invention relates to a method for screening one or more factors for involvement in T-cell therapeutic function, comprising
a. provision of regulatory or pathogenic T-cells isolated from a donor mouse,
b. modification of said isolated T-cells via transfection of one or more nucleic acid molecules encoding a MOG-binding TCR, said MOG-binding TCR comprising the MOG-binding TCR beta chain of claim 1 and a MOG-binding TCR α chain that forms a MOG-binding TCR with the TCR beta chain of claim 1,
c. treatment of modified T-cells with a factor to be investigated, and
d. in vitro assay for T-cell therapeutic function.

Also disclosed herein is that:
- the nucleic acid molecule(s) encoding the MOG-binding TCR β chain and α chain of step b. are isolated from the transgenic mouse as disclosed herein.

In one embodiment the invention is characterised in that:
- the T-cells are isolated directly from the transgenic mouse as described herein and step b. is not carried out.

In one embodiment the method is characterised in that the factor to be investigated is a candidate compound, preferably selected from a small molecule, antibody, anti-sense nucleic acid molecule, cytokine and/or peptide.

In one embodiment the method is characterised in that step c. is replaced by using a genetically modified donor mouse in step a., whereby the genetic modification of the donor mouse is the factor to be screened for involvement in T-cell therapeutic function.

Also disclosed herein is that:
- the in vivo assay of step d. comprises administration of modified T-cells to a mouse with an autoimmune disease or a model thereof such as experimental autoimmune encephalomyelitis (EAE), whereby the therapeutic effect of the modified T-cells is measured.

In one embodiment the invention is characterised in that:
- the in vitro test of step d. comprises testing for any kind of T-cell function, such as testing for cytokine production in cell culture.

In one embodiment the method is characterised in that the isolated T-cells of step a. are
- regulatory T-cells (Treg) characterised by the markers CD4+, CD25+, and preferably Fox3p+, and is optionally additionally characterised by expression of markers IL-10, TGF-β and/or IL-35, or
- pathogenic T-cells (Tconv) characterised by the markers CD4+, CD25-, and preferably Fox3p-, and is optionally additionally characterised by expression of markers IFN-gamma, GM-CSF, IL-17, IL-21, IL-22, TNF, IL-6, lymphotoxin, granzyme, and/or perforin.

A further aspect of the invention relates to the use of a transgenic mouse, T-cell receptor (TCR), or a cell isolated from the transgenic mouse as described herein, as a model for investigation of T-cell function in an immune disease.

A further aspect of the invention relates to the use of a transgenic mouse, T-cell receptor (TCR), or a cell isolated from the transgenic mouse or a method as described herein, for the identification of drug targets in immune disease.

### DETAILED DESCRIPTION OF THE INVENTION

We have developed a novel TCR transgenic mouse (called Kaa mouse) for investigating the pathogenic function of Tconv cells and the protective roles of Treg cells in experimental autoimmune encephalomyelitis (EAE), which is the primary pre-clinical model for relapsing-remitting multiple sclerosis (RR-MS).

The invention enables utilization of the Kaa mouse and derived TCR to investigate the mechanisms controlling the protective function of Treg cells, and the pathogenic role of Tconv cells during autoimmune disease, thereby providing a fast forward approach to drug target discovery:

### Investigation of the genes/pathways controlling the protective function of Treg cells:

The Kaa mouse can be backcrossed to genetically modified mice to investigate the effect of the selected gene on the protective function of the Kaa Treg cells. This could involve adoptive transfer of mutant CD4+CD25+ T cells from the resulting mutant Kaa mouse into recipient animals.

Kaa-derived TCR can be introduced into polyclonal CD4+CD25+ T cells from genetically modified or wild type mice to investigate the effect of the selected gene on the protective function of Treg cells. This bypasses any need for breeding and allows direct investigation of the effect of selected mutations on the protective function of the Treg cells. It would also be possible to subject the CD4+CD25+ T cells to various treatments during their culture in vitro, such as screening with libraries of small molecules or RNAi, to assess how these treatments affect the protective functions of these T cells in adoptive transfer experiments.

### Investigation of the genes/pathways controlling the pathogenic function of Tconv cells:

The Kaa mouse can be backcrossed to genetically modified mice to investigate the effect of the selected gene on the pathogenic function of the Kaa Tconv cells. This could involve adoptive transfer of mutant CD4+CD25- T cells from the resulting mutant Kaa mouse into recipient animals. It is also possible to subject the CD4+CD25- T cells to various treatments during their culture in vitro to assess how these treatments affect the pathogenic function of the Tconv cells in adoptive transfer experiment.

Kaa-derived TCR can be introduced into polyclonal CD4+CD25- T cells from wild-type mice to make encephalitogenic T cells that will provoke disease upon adoptive transfer into recipient mice. Such a system could then be used with CD4+CD25- T cells from gene-modified mice to investigate the effect of this gene on the pathogenic function of T cells. This would bypass any need for breeding and allow direct investigation of the effect of selected mutations on the pathogenic function of the Tconv cells. It would also be possible to subject the CD4+CD25- T cells to various treatments during their culture in vitro to assess how these treatments affect the pathogenic function of the Tconv cells in adoptive transfer experiment.

In conclusion, the Kaa system (mouse and TCR molecules) provides an extremely powerful environment for the rapid identification of new drug targets, and the testing of novel therapies for autoimmune diseases, as well as other types of immune-mediated diseases.

The invention therefore relates to the Kaa transgenic mouse as well as all Kaa derived TCR molecules, in a preferred embodiment TCR that contain the TCR beta chain amino acid sequence and any given TCR alpha chain, provided that the TCR is MOG-binding, and the application of these reagents to dissect the mechanisms controlling the protective function of Treg cells (e.g. CD4+Foxp3+ T cells, but also other subsets of regulatory T cells), and the pathogenic functions of Tconv cells (e.g. CD4+Foxp3- T cells, but also other subsets of T cells).

The invention therefore also relates to a method for screening one or more factors for involvement in T-cell therapeutic function. T-cells exhibit therapeutic functions with respect to various immune diseases, preferably autoimmune diseases. The method of the invention in a preferred embodiment involves administration of modified T-cells to a mouse with an autoimmune disease or a model thereof such as experimental autoimmune encephalomyelitis (EAE), whereby the therapeutic effect of the modified T-cells is measured. The therapeutic effect can be measured by assessing the degree, severity and/or progression of the disease before and after treatment with the modified T-cells in a quantitative manner. Therefore immune disease models may be used that are equivalent to relevant human immune diseases, in order to provide in a model system a method of examining potential therapeutic effects of the modified T-cells.

An "immune disease" is defined as any medical condition or disorder where the immune system plays a role in the pathology of the disease, in its origin and/or progression. Examples of immune diseases are therefore autoimmune diseases, allergies, inflammation disorders, transplantation-related disorders and/or type 2 diabetes.

An "autoimmune disorder" or "autoimmune disease" herein is a disease or disorder arising from an immune response directed against an individual's own tissues or a co-segregate or manifestation thereof or resulting condition therefrom. Examples of autoimmune diseases or disorders include, but are not limited to arthritis (rheumatoid arthritis, juvenile-onset rheumatoid arthritis, osteoarthritis, psoriatic arthritis, and ankylosing spondylitis), psoriasis, dermatitis including atopic dermatitis, chronic idiopathic urticaria, including chronic autoimmune urticaria, polymyositis/ dermatomyositis, toxic epidermal necrolysis, scleroderma (including systemic scleroderma), sclerosis such as progressive systemic sclerosis, inflammatory bowel disease (IBD) (for example, Crohn's disease, ulcerative colitis, autoimmune inflammatory bowel disease), pyoderma gangrenosum, erythema nodosum, primary sclerosing cholangitis, episcleritis), respiratory distress syndrome, including adult respiratory distress syndrome (ARDS), meningitis, IgE-mediated diseases such as anaphylaxis and allergic and atopic rhinitis, encephalitis such as Rasmussen's encephalitis, uveitis or autoimmune uveitis, colitis such as microscopic colitis and collagenous colitis, glomerulonephritis (GN) such as membranous GN (membranous nephropathy), idiopathic membranous GN, membranous proliferative GN (MPGN), including Type I and Type II, and rapidly progressive GN, allergic conditions, allergic reaction, eczema, asthma, conditions involving infiltration of T cells and chronic inflammatory responses, atherosclerosis, autoimmune myocarditis, leukocyte adhesion deficiency, systemic lupus erythematosus (SLE) such as cutaneous SLE, subacute cutaneous lupus erythematosus, lupus (including nephritis, cerebritis, pediatric, non-renal, discoid, alopecia), juvenile onset (Type I) diabetes mellitus, including pediatric insulin-dependent diabetes mellitus (IDDM), adult onset diabetes mellitus (Type II diabetes), multiple sclerosis (MS) such as spino-optical MS, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitis (including large vessel vasculitis (including polymyalgia rheumatica and giant cell (Takayasu's) arteritis), medium vessel vasculitis (including Kawasaki's disease and polyarteritis nodosa), CNS vasculitis, systemic necrotizing vasculitis, and ANCA-associated vasculitis, such as Churg-Strauss vasculitis or syndrome (CSS)), temporal arteritis, aplastic anemia, Coombs positive anemia, Diamond Blackfan anemia, hemolytic anemia or immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, antigen-antibody complex mediated diseases, anti-glomerular basement membrane disease, antiphospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous, pemphigus (including vulgaris, foliaceus, and pemphigus mucus-membrane pemphigoid), autoimmune polyendocrinopathies, Reiter's disease, immune complex nephritis, chronic neuropathy such as IgM polyneuropathies or IgMmediated neuropathy, thrombocytopenia (as developed by myocardial infarction patients, for example), including thrombotic thrombocytopenic purpura (TTP) and autoimmune or immune-mediated thrombocytopenia such as idiopathic thrombocytopenic purpura (ITP) including chronic or acute ITP, autoimmune disease of the testis and ovary including autoimune orchitis and oophoritis, primary hypothyroidism, hypoparathyroidism, autoimmune endocrine diseases including thyroiditis such as autoimmune thyroiditis, chronic thyroiditis (Hashimoto's thyroiditis), or subacute thyroiditis, autoimmune thyroid disease, idiopathic hypothyroidism, Addison's disease, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis, myasthenia gravis, cerebellar degeneration, limbic and/or brainstem encephalitis, neuromyotonia, opsoclonus or opsoclonus myoclonus syndrome (OMS), and sensory neuropathy, Sheehan's syndrome, autoimmune hepatitis, chronic hepatitis, lupoid hepatitis, chronic active hepatitis or autoimmune chronic active hepatitis, lymphoid interstitial pneumonitis, bronchiolitis obliterans (non-transplant) vs NSIP, GuillainBarre syndrome, Berger's disease (IgA nephropathy), primary biliary cirrhosis, celiac sprue (gluten enteropathy), refractory sprue, dermatitis herpetiformis, cryoglobulinemia, amyotrophic lateral sclerosis (ALS; Lou Gehrig's disease), coronary artery disease, autoimmune inner ear disease (AIED) or autoimmune hearing loss, opsoclonus myoclonus syndrome (OMS), polychondritis such as refractory polychondritis, pulmonary alveolar proteinosis, amyloidosis, giant cell hepatitis, scleritis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine ophthalmopathy, uveoretinitis, autoimmune hepatological disorder, fibromyalgia, multiple endocrine failure, Schmidt's syndrome, adrenalitis, gastric atrophy, presenile dementia, demyelinating diseases, Dressler's syndrome, alopecia areata, CREST syndrome (calcinosis, Raynaud's phenomenon, esophageal dysmotility, sclerodactyly, and telangiectasia), male and female autoimmune infertility, ankylosing spondolytis, mixed connective tissue disease, Chagas' disease, rheumatic fever, recurrent abortion, farmer's lung, erythema multiforme, post-cardiotomy syndrome, Cushing's syndrome, bird-fancier's lung, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, and giant cell polymyalgia. The treatment of autoimmune disorders via immune suppression is therefore one embodiment of the present invention.

As used herein, the term "allergy" or "allergies" or "allergic reaction" or "allergic response" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a major role in conditions such as asthma. In some people, severe allergies to environmental or dietary allergens or to medication may result in life-threatening anaphylactic reactions and potentially death. The treatment of allergies via immune suppression is therefore one embodiment of the present invention.

As used herein, the term "inflammation" or "inflammation disorder" refers by way of example to type 1 diabetes (e.g.diabetic nephropathy), rheumatoid arthritis, osteoarthritis, polyarthritis, gout, systemic lupus erythematosus, scleroderma, Sjorgen's syndrome, poly- and dermatomyositis, vasculitis, tendonitis, synovitis, bacterial endocarditis, osteomyelitis, psoriasis, pneumonia, fibrosing alveolitis, chronic bronchitis, chronic obstructive pulmonary disease (COPD), bronchiectasis, emphysema, silicosis, tuberculosis, ulcerative colitis and Crohn's disease.chronic inflammatory demyelinating polyradiculoneuropathy, chronic inflammatory demyelinating polyneuropathy, multiple sclerosis, Guillan-Barre Syndrome and myasthemia gravis, mastitis, laminitis, laryngitis, chronic cholecystitis, Hashimoto's thyroiditis, and including chronic inflammatory renal disease. In a further embodiment of the present invention said diorder is an inflammatory renal disease, such as glomerulonephritis, crescentic glomerulonephritis, lupus nephritis, ANCA-associated glomerulonephritis, focal and segmental necrotizing glomerulonephritis, IgA nephropathy, membranoproliferative glomerulonephritis, cryoglobulinaemia and tubulointerstitial nephritis and tubulointerstitial nephritis.

Transplantation-related disorders relate to any medical condition surrounding rejection of transplanted material, or an immune response caused by transplantation of tissue to a recipient subject.

The method of the present invention therefore allows interrogation of T-cell function and therapeutic effect (and especially with regard to the role of other genes or factors to be investigated and their role on T-cell function during adoptive T-cell transfer), with regard to enhancing Treg function or repressing Tconv function, in any of the above-mentioned medical conditions. The invention therefore relates to methods for screening substances for use in the treatment of the above-mentioned conditions.

In some embodiments the TCR of the present invention are characterized by concrete sequences of the alpha and beta chains. Also disclosed herein are amino acid sequences with more than 80% sequence identity, preferably more than 90% sequence identity, to those concrete sequences provided by SEQ ID No. 1, 2 and in Table A, for example, 80, 81, 82, 83, 84, 85, 86, 87, 88, 89, 90, 91, 92, 93, 94, 95, 96, 97, 98, 99% sequence identity, that maintain MOG-binding. Sequence identity is a measure of sequence similarity between two sequences. The sequence variants of the present invention as described herein are functionally analogous, whereby functionally analogous sequences are those that although exhibiting some difference in amino acid sequence exhibit the same key function in the context of the present invention, namely MOG-binding. Functionally analogous sequences can be tested by one skilled in the art without inventive effort in light of the information provided within the application, or by using other approaches known to those skilled in the art, for example any physical interaction studies or corresponding in vivo or in vitro approaches that provide information on whether the sequence variant maintains MOG-binding in a similar manner to those sequences listed explicitly.

### Sequences of the Invention:

**SEQ ID No 1:** GETGNNYAEQ
**SEQ ID No 2:**

**Table A - Amino acid sequences of 38 preferred MOG reactive TCR α chains, showing V-J rearrangements, with CDR3 sequences indicated. The constant region, which is common to all TCR α chains, is not included. All these TCR α chains form MOG-reactive TCR upon association with the Kaa TCR β chain.**

| 1) Sequence ID: N12-0300 |
|---|
| V-region: TRAV13D-1*02 |
| J-region: TRAJ17*01 |
| CDR3: CAFFNSAGNKLTF (SEQ ID No. 3) |
| V-J region AA sequence: |
| |

| 2) Sequence ID: N14-0089 |
|---|
| V-region: TRAV4N-3*01 |
| J-region: TRAJ37*01 |
| CDR3: CAAELTGNTGKLIF (SEQ ID No. 5) |
| V-J region AA sequence: |
| |

| 3) Sequence ID: N14-0435 |
|---|
| V-region: TRAV4D-3*03 |
| J-region: TRAJ13*01 |
| CDR3: CAAEAGSGTYQRF (SEQ ID No. 7) |
| V-J region AA sequence: |
| |

| 4) Sequence ID: N14-1041 |
|---|
| V-region: TRAV4N-3*01 |
| J-region: TRAJ13*01 |
| CDR3: CAAEAGSGTYQRF (SEQ ID No. 9) |
| V-J region AA sequence: |
| |

| 5) Sequence ID: N14-2140 |
|---|
| V-region: TRAV4D-3*03 |
| J-region: TRAJ39*01 |
| CDR3: CAADNAGAKLTF (SEQ ID No. 11) |
| V-J region AA sequence: |
| |

| 6) Sequence ID: N5-127 |
|---|
| V-region: TRAV4D-3*03 |
| J-region: TRAJ27*01 |
| CDR3: CAARNTNTGKLTF (SEQ ID No. 13) |
| V-J region AA sequence: |
| |

| 7) Sequence ID: N5-135 |
|---|
| V-region: TRAV13N-1*01 |
| J-region: TRAJ42*01 |
| CDR3: CAMGGGSNAKLTF (SEQ ID No. 15) |
| V-J region AA sequence: |
| |

| 8) Sequence ID: N5-197 |
|---|
| V-region: TRAV4D-3*03 |
| J-region: TRAJ50*01 |
| CDR3: CAATASSSFSKLVF (SEQ ID No. 17) |
| V-J region AA sequence: |
| |

| 9) Sequence ID: N5-307 |
|---|
| V-region: TRAV4N-3*01 |
| J-region: TRAJ21*01 |
| CDR3: CAAGNYNVLYF (SEQ ID No. 19) |
| V-J region AA sequence: |
| |

| 10) Sequence ID: N5-575 |
|---|
| V-region: TRAV4D-3*03 |
| J-region: TRAJ22*01 |
| CDR3: CAADSSGSWQLIF (SEQ ID No. 21) |
| V-J region AA sequence: |
| |

| 11) Sequence ID: N8-309 |
|---|
| V-region: TRAV4D-3*03 |
| J-region: TRAJ21*01 |
| CDR3: CAAGNYNVLYF (SEQ ID No. 23) |
| V-J region AA sequence: |
| |

| 12) Sequence ID: N8-68 |
|---|
| V-region: TRAV4N-3*01 |
| J-region: TRAJ27*01 |
| CDR3: CAARNTNTGKLTF (SEQ ID No. 25) |
| V-J region AA sequence: |
| |

| 13) Sequence ID: T10-78 |
|---|
| V-region: TRAV4D-3*03 |
| J-region: TRAJ17*01 |
| CDR3: CAPTNSAGNKLTF (SEQ ID No. 27) |
| V-J region AA sequence: |
| |
| |

| 14) Sequence ID: T11-1722 |
|---|
| V-region: TRAV4D-3*03 |
| J-region: TRAJ47*01 |
| CDR3: CAADYANKMIF (SEQ ID No. 29) |
| V-J region AA sequence: |
| |

| 15) Sequence ID: T11-2161 |
|---|
| V-region: TRAV7-2*02 |
| J-region: TRAJ27*01 |
| CDR3: CAASKANTGKLTF (SEQ ID No. 31) |
| V-J region AA sequence: |
| |

| 16) Sequence ID: T12-0082 |
|---|
| V-region: TRAV4N-3*01 |
| J-region: TRAJ26*01 |
| CDR3: CAAANYAQGLTF (SEQ ID No. 33) |
| V-J region AA sequence: |
| |

| 17) Sequence ID: T12-0364 |
|---|
| V-region: TRAV4D-3*03 |
| J-region: TRAJ37*01 |
| CDR3: CAAPGNTGKLIF (SEQ ID No. 35) |
| V-J region AA sequence: |
| |

| 18) Sequence ID: T12-0578 |
|---|
| V-region: TRAV4N-3*01 |
| J-region: TRAJ15*01 |
| CDR3: CAAQGGRALIF (SEQ ID No. 37) |
| V-J region AA sequence: |
| |

| 19) Sequence ID: T12-0832 |
|---|
| V-region: TRAV4D-3*03 |
| J-region: TRAJ16*01 |
| CDR3: CAATSSGQKLVF (SEQ ID No. 39) |
| V-J region AA sequence: |
| |

| 20) Sequence ID: T12-1359 |
|---|
| V-region: TRAV4D-3*03 |
| J-region: TRAJ31*01 |
| CDR3: CAAEAGNNRIFF (SEQ ID No. 41) |
| V-J region AA sequence: |
| |

| 21) Sequence ID: T12-2042 |
|---|
| V-region: TRAV4D-3*03 |
| J-region: TRAJ15*01 |
| CDR3: CAAQGGRALIF (SEQ ID No. 43) |
| V-J region AA sequence: |
| |

| 22) Sequence ID: T13-0964 |
|---|
| V-region: TRAV4D-3*03 |
| J-region: TRAJ33*01 |
| CDR3: CAASNYQLIW (SEQ ID No. 45) |
| V-J region AA sequence: |
| |

| 23) Sequence ID: T13-1126 |
|---|
| V-region: TRAV4N-3*01 |
| J-region: TRAJ27*01 |
| CDR3: CAAHNTNTGKLTF (SEQ ID No. 47) |
| V-J region AA sequence: |
| |

| 24) Sequence ID: T14-0058 |
|---|
| V-region: TRAV4N-3*01 |
| J-region: TRAJ17*01 |
| CDR3: CAAGNSAGNKLTF (SEQ ID No. 49) |
| V-J region AA sequence: |
| |

| 25) Sequence ID: T5-09 |
|---|
| V-region: TRAV4D-3*03 |
| J-region: TRAJ37*01 |
| CDR3: CAAEVAGNTGKLIF (SEQ ID No. 51) |
| V-J region AA sequence: |
| |

| 26) Sequence ID: T5-110 |
|---|
| V-region: TRAV4-3*01 |
| J-region: TRAJ42*01 |
| CDR3: CAAEPARSNAKLTF (SEQ ID No. 53) |
| V-J region AA sequence: |
| |

| 27) Sequence ID: T5-15 |
|---|
| V-region: TRAV4D-3*03 |
| J-region: TRAJ37*01 |
| CDR3: CAAELTGNTGKLIF (SEQ ID No. 55) |
| V-J region AA sequence: |
| |

| 28) Sequence ID: T5-35 |
|---|
| V-region: TRAV4D-3*03 |
| J-region: TRAJ34*02 |
| CDR3: CAASNTNKVVF (SEQ ID No. 57) |
| V-J region AA sequence: |
| |

| 29) Sequence ID: T5-36 |
|---|
| V-region: TRAV4-3*01 |
| J-region: TRAJ42*01 |
| CDR3: CAAEPARSNAKLTF (SEQ ID No. 59) |
| V-J region AA sequence: |
| |

| 30) Sequence ID: T6-106 |
|---|
| V-region: TRAV7-2*02 |
| J-region: TRAJ27*01 |
| CDR3: CAASGANTGKLTF (SEQ ID No. 61) |
| V-J region AA sequence: |
| |

| 31) Sequence ID: T6-348 |
|---|
| V-region: TRAV4D-3*03 |
| J-region: TRAJ31*01 |
| CDR3: CAAGNSNNRIFF (SEQ ID No. 63) |
| V-J region AA sequence: |
| |

| 32) Sequence ID: T6-71 |
|---|
| V-region: TRAV7-2*02 |
| J-region: TRAJ52*01 |
| CDR3: CAASGANTGKLTF (SEQ ID No. 65) |
| V-J region AA sequence: |
| |
| |

| 33) Sequence ID: T6-91 |
|---|
| V-region: TRAV4D-3*03 |
| J-region: TRAJ37*01 |
| CDR3: CAAEITGNTGKLIF (SEQ ID No. 67) |
| V-J region AA sequence: |
| |

| 34) Sequence ID: T7-701 |
|---|
| V-region: TRAV4D-3*03 |
| J-region: TRAJ30*01 |
| CDR3: CAAPVTNAYKVIF (SEQ ID No. 69) |
| V-J region AA sequence: |
| |

| 35) Sequence ID: T7-925 |
|---|
| V-region: TRAV4D-3*03 |
| J-region: TRAJ42*01 |
| CDR3: CAADGGSNAKLTF (SEQ ID No. 71) |
| V-J region AA sequence: |
| |

| 36) Sequence ID: T8-1310 |
|---|
| V-region: TRAV4N-3*01 |
| J-region: TRAJ30*01 |
| CDR3: CAAPVTNAYKVIF (SEQ ID No. 73) |
| V-J region AA sequence: |
| |

| 37) Sequence ID: T9-154 |
|---|
| V-region: TRAV4D-3*03 |
| J-region: TRAJ31*01 |
| CDR3: CAAASNNRIFF (SEQ ID No. 75) |
| V-J region AA sequence: |
| |

| 38) Sequence ID: T9-360 |
|---|
| V-region: TRAV4D-3*03 |
| J-region: TRAJ26*01 |
| CDR3: CAANYAQGLTF (SEQ ID No. 77) |
| V-J region AA sequence: |
| |

### Figures

The figures provided herein represent examples of particular embodiments of the invention and are not intended to limit the scope of the invention. The figures are to be considered as providing a further description of possible and potentially preferred embodiments that enhance the technical support of one or more non-limiting embodiments.

### Short description of the figures

**Figure 1****:** Enrichment for Vβ8.2-expressing cells in Tconv and Treg cells in CNS of mice with EAE.
**Figure 2****:** The Kaa mouse develops an attenuated form of EAE following immunization with MOG(35-55).
**Figure 3****:** Kaa T cells are highly encephalitogenic.
**Figure 4****:** CD4⁺CD25⁺ Kaa T cells protect from EAE.
**Figure 5****:** Distribution of repeated CDR3 in Treg and Tconv cells.
**Figure 6****:** IL-2 production by T cell lines expressing TCR derived from CNS Tconv cells or from CNS Treg cells.
**Figure 7****:** Re-directed Treg cells fully protect recipient mice from EAE.

### Detailed description of the figures

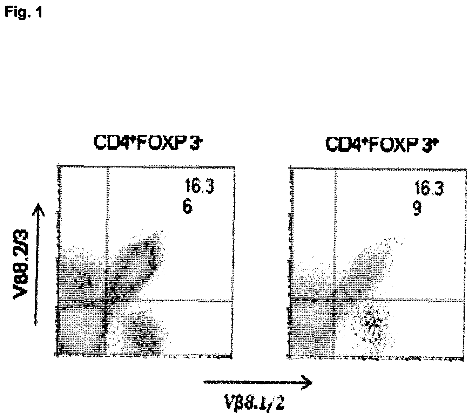

**Figure 1****:** Enrichment for Vβ8.2-expressing cells in Tconv and Treg cells in CNS of mice with EAE. CNS-infiltrating cells were isolated from C57BL/6 mice at day 21 after EAE induction with MOG(35-55), and stained with anti-CD4, anti-Vβ8.1/8.2, anti-Vβ8.2/8.3, and anti-Foxp3 antibodies. The plots are gated on CNS CD4⁺Foxp3⁻ (Tconv), and CD4⁺Foxp3⁺ (Treg) cells, respectively. In spleen, only 10% of CD4⁺Foxp3⁻ and CD4⁺Foxp3⁺ cells were Vβ8.2⁺ (not shown).

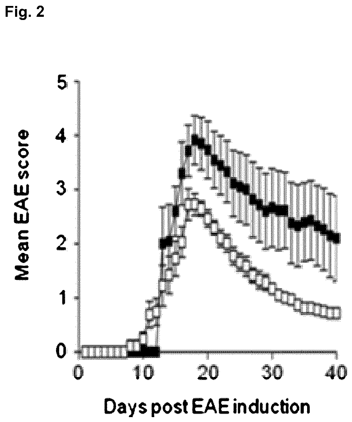

**Figure 2****:** The Kaa mouse develops an attenuated form of EAE following immunization with MOG(35-55). EAE was induced in Kaa mouse (white square) and C57BL/6 mice (black square). Data show mean clinical score and standard error mean (s.e.m).

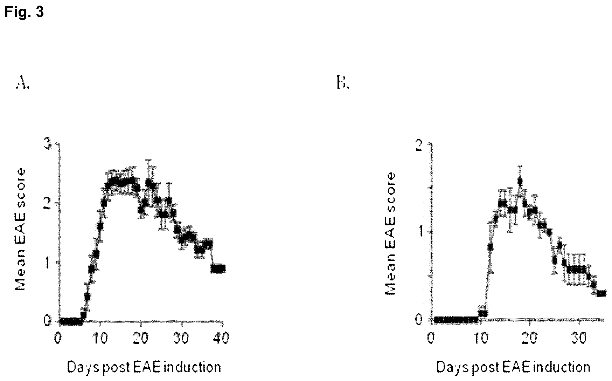

**Figure 3****:** Kaa T cells are highly encephalitogenic. A. Kaa mice were immunized with MOG(35-55) in complete Freund's adjuvant via the subcutaneous route. At day 8, draining lymph node cells were harvested and re-stimulated for 3 days in presence of MOG(35-55), IL-2, IL-12, an IL-18, before adoptive transfer into naive C57BL/6 mice (6 10⁶ cells/mouse). Data show mean EAE scores of C57BL/6 recipient. B. Cells from lymph nodes and spleens of naïve Kaa mice were isolated and depleted of CD25⁺ cells before being activated *in vitro* for 3 days with MOG(35-55), IL-2, IL-12, and IL-18. 10 10⁶ blasts were injected into naïve C57BL/6 mice. Data show mean EAE score of C57BL/6 recipient. Error bars show s.e.m. All recipients were treated with pertussis toxin on days 0 and 2.

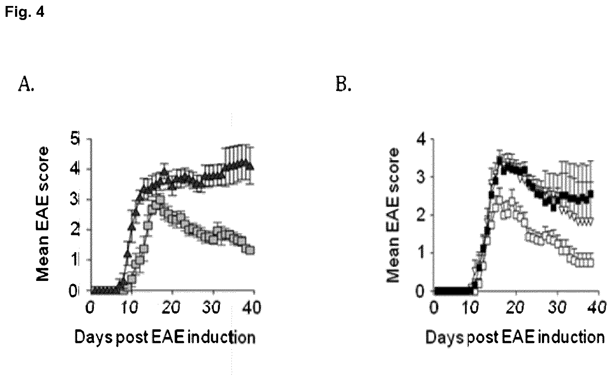

**Figure 4****:** CD4⁺CD25⁺ Kaa T cells protect from EAE. A) EAE was induced on day 0 by immunization with MOG(35-55) in Kaa mice pre-treated 3 days earlier with 200 µg anti-CD25 antibody (PC61) (dark triangles), or in untreated Kaa mice (grey squares). B) C57BL/6 mice received 3 10⁶ CD4⁺CD25⁺ T cells from naive C57BL/6 mice (white triangles) or from naïve Kaa mice (white squares) one day before EAE induction by immunization with MOG(35-55). Control C57BL/6 mice did not receive any cells (black squares). Data show mean clinical score and s.e.m.

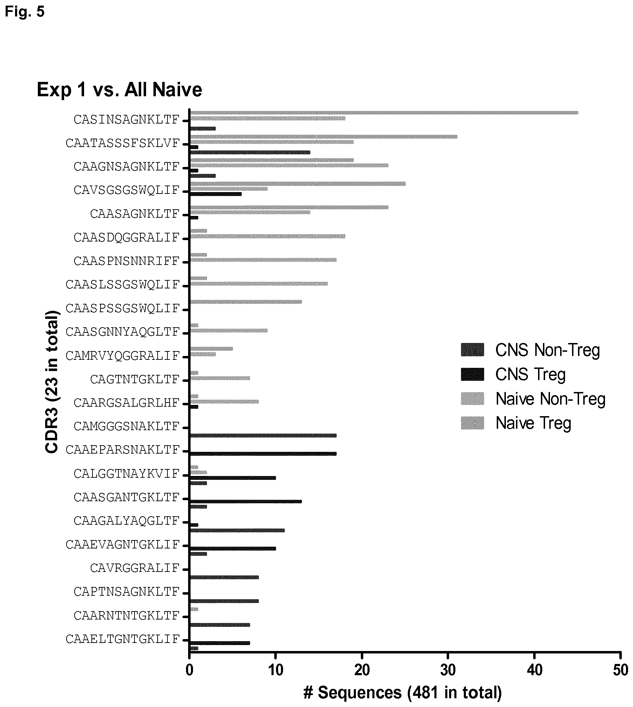

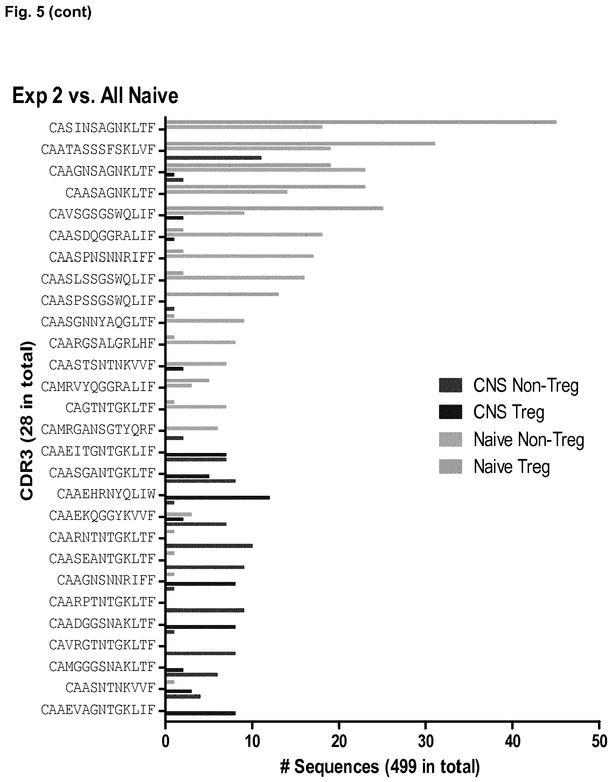

**Figure 5****:** Distribution of repeated CDR3 in Treg and Tconv cells. Data show the occurrence of CDR3 sequences found at least 8 times among the complete set of obtained sequences in Treg and Tconv (non-Treg) cells from CNS of EAE mice or from secondary lymphoid organs of naïve mice.

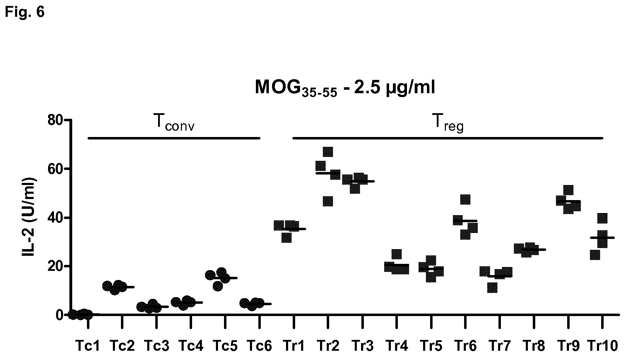

**Figure 6****:** IL-2 production by T cell lines expressing TCR derived from CNS Tconv cells (blue circles) or from CNS Treg cells (red squares) after stimulation with MOG(35-55) at 2.5 µg/ml. For each TCR, 4 independent cell lines were tested. All T cell lines did not secrete any IL-2 in the absence of MOG, but produced IL-2 with 40 µg/ml MOG (data not shown).

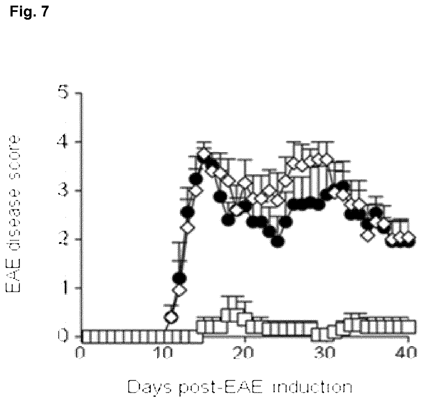

**Figure 7****:** Re-directed Treg cells fully protect recipient mice from EAE. C57BL/6 mice received 8x10⁵ polyclonal Treg transduced with a TCR from MOG-reactive Treg cells (white square), or 8x10⁵ polyclonal Treg transduced with a MOCK vector (white diamond) on day -1, or were left untreated (black circle). EAE was induced on day 0 by immunization with MOG(35-55) in adjuvant.

### EXAMPLES

The examples provided herein represent practical support for particular embodiments of the invention and are not intended to limit the scope of the invention. The examples are to be considered as providing a further description of possible and potentially preferred embodiments that demonstrate the relevant technical working of one or more non-limiting embodiments.

### Example 1 - Development of the Kaa mouse to investigate pathogenic and protective functions of CD4⁺ T cells in CNS autoimmunity

We have developed a novel TCR transgenic mouse (called Kaa mouse) for investigating the pathogenic function of Tconv cells and the protective roles of Treg cells in experimental autoimmune encephalomyelitis (EAE), which is the primary pre-clinical model for relapsing-remitting multiple sclerosis (RR-MS). EAE is a T cell mediated autoimmune disease that leads to development of inflammatory demyelinating disease lesions in the central nervous system (CNS). This disease can be induced in mice of the C57BL/6 background, on which most genetic alteration of the mouse genome have been produced, using myelin oligodendrocyte glycoprotein (MOG) as autoantigen. Of note, MOG is also a major candidate autoantigen in RR-MS.

To select a relevant MOG-reactive TCR to produce the Kaa mouse, we considered the fact that the T cells acting as drivers of autoimmune pathogenesis often carry particular TCR, which are expressed by some antigen-reacting T cells in all individuals of identical MHC haplotype, and are therefore called "public TCR". We have previously identified the public TCR rearrangements of the anti-MOG CD4⁺ T cell response during EAE induced in C57BL/6 mice. The public TCRβ sequence of this CD4⁺ T cell response is characterized by a Vβ8.2-Jβ2.1 rearrangement and a GETGNNYAEQ CDR3 sequence. This TCR sequence is expressed by T_{H}1 and Treg cells in CNS of wild-type mice with EAE, suggesting that this rearrangement is expressed by both encephalitogenic and protective T cells (Figure 1).

We have obtained a T cell hybridoma, which expresses this particular TCRβ chain, and reacts against MOG. We could clone the TCRβ chain from this hybridoma, and generated at the DRFZ the transgenic Kaa mouse expressing the public TCRβ chain under the control of a CD2 promoter on the C57BL/6 background.

The transgenic mouse was generated using classical techniques, via injection of transgenic expression construct into mouse eggs, before transplantation to the uterus of a surrogate mother and appropriate screening for transgenic offspring. Such techniques are known to one skilled in the art.

To quantify the number of MOG-reactive CD4⁺ T cells in naive Kaa mice, we measured the numbers of CD4⁺ cells that up-regulated the activation marker CD154 (CD40L), which identifies antigen-reactive CD4⁺ T cells (29). In cultures of Kaa splenocytes stimulated with 40 µg/ml MOG(35-55) for 6 hours, about 2% of Kaa CD4⁺ T cells up-regulated CD154, while no up-regulation of CD154 was detected on CD4⁺ T cells in cultures of C57BL/6 cells (data not shown). We conclude from these results that Kaa mice contain an elevated frequency of MOG-reactive Tconv cells compared to C57BL/6 mice, as expected.

### Example 2 - The Kaa mouse develops a self-limiting EAE upon immunization with MOG(35-55):

We immunized Kaa and C57BL/6 mice with MOG(35-55) in adjuvant, and compared the course of EAE in these two types of mice. Remarkably, Kaa mice developed a milder form of EAE than C57BL/6 mice and recovered efficiently from the disease, even though they had an increased number of MOG-reactive CD4⁺ T cells (Figure 2).

### Example 3 - CD4⁺CD25⁻ T cells from Kaa mice are highly encephalitogenic

To evaluate the encephalitogenic potential of Kaa CD4⁺ T cells, we performed three types of adoptive transfer experiments.

First, we adoptively transferred naïve CD4⁺CD25⁻ Kaa T cells into naive C57BL/6 mice that were subsequently immunized with MOG(35-55) on the next day. Mice that had received Kaa CD4⁺CD25⁻ T cells developed a more severe EAE than C57BL/6 mice that received CD4⁺CD25⁻ T cells from C57BL/6 mice (data not shown).

Second, we took lymph node cells from Kaa mice immunized with MOG(35-55) 8 days earlier, and re-stimulated them for 3 days *in vitro*, before adoptively transferring them into naïve C57BL/6 recipient mice (Figure 3A). Such transfer of activated Kaa cells resulted in development of EAE in recipient mice (Figure 3A).

Third, we could induce EAE in naïve C57BL/6 mice by adoptive transfer of naïve CD4⁺CD25⁻ Kaa T cells after pre-activation of these cells *in vitro* for 72 hours with MOG, IL-2, IL-12, and IL-18 (Figure 3B). From these observations we conclude that Kaa CD4⁺ T cells are highly encephalitogenic. Importantly, these data show that the Kaa mouse is a unique model to dissect the mechanisms controlling the differentiation of naïve autoreactive CD4⁺ T cells into disease-causing pathogenic effector T cells. For instance, the Kaa mouse could be crossed to any genetically modified mouse strain to test the function of selected genes in the pathogenic function of autoreactive T cells. It would also be possible to treat cells with pharmacological compounds (or small inhibitory RNA) during the 72h activation period *in vitro* to test the effect of these substances or genes on the encephalitogenic function of the cells after transfer in recipient animals.

### Example 4 - Kaa mice contain an elevated frequency of MOG-reactive CD4⁺Foxp3⁺ Treg

We then assessed whether Kaa mice contain an increased number of MOG-reactive Treg cells, which could explain why they only develop a mild form of EAE upon immunization with MOG, compared to C57BL/6 mice.

The first evidence that Kaa Treg cells provide protection from EAE was that Kaa mice treated with an anti-CD25 antibody, which reduces numbers and functions of Treg cells, developed a severe chronic form of EAE upon immunization with MOG(35-55) (Figure 4A).

The second evidence of the protective role of Kaa Treg cells was that adoptive transfer of CD4⁺CD25⁺ T cells from naïve Kaa mice into recipient C57BL/6 mice resulted in an attenuation of the EAE course in C57BL/6 mice, while transfer of CD4⁺CD25⁺ T cells from C57BL/6 mice had no effect (Figure 4B). This result confirms the protective function of Kaa CD4⁺CD25⁺ Treg cells. It also indicates that Treg cells protect from EAE in an antigen-specific manner, implying that Tregs require TCR triggering to achieve suppression *in vivo*.

In addition, we confirmed that Kaa mice possess elevated amounts of MOG-reactive Treg cells using *in vitro* experiments. First, the proliferative response of Kaa CD4⁺ T cells stimulated with MOG *in vitro* was highly enhanced by prior elimination of CD25⁺ T cells (data not shown). Second, CD4⁺CD25⁺ Kaa Treg cells suppressed the proliferation of OT-II T cells (which express an ovalbumin-reactive TCR) activated with ovalbumin in a MOG-dependent manner *in vitro* (data not shown). Taken together, our *in vivo* and *in vitro* data demonstrate that the Kaa mouse contains elevated numbers of MOG-reactive Treg cells.

### Example 5 - Characterization of the TCRα repertoires of Treg and Tconv cells found in CNS of Kaa mice at peak of EAE

We then used the Kaa mouse to compare the TCR repertoires from pathogenic MOG-reactive Tconv cells and protective MOG-reactive Treg cells. To this end, we first sought to clone the TCR carried by MOG-reactive Tconv and Treg cells present in the CNS of Kaa mice at the peak of EAE. For this purpose, the Kaa mouse was backcrossed on a TCRβ^{+/-}TCRα^{+/-}Foxp3.eGFP background.

Kaa⁺TCRβ^{+/-}TCRα^{+/-}Foxp3.eGFP mice were then immunized with MOG, and CD4⁺GFP⁺ (Treg) cells and CD4⁺GFP⁻ (Tconv) cells were isolated from CNS at peak of EAE severity. The RNA from CNS Treg and Tconv cells were then extracted and subjected to 5' RACE PCR reactions using a primer annealing to the 3' end of the TCR Cα region. This technology enabled us to clone the full-length TCRα sequences of CNS Treg and Tconv cells including their complete 3'Cα segment and their entire 5'Vα coding region without any knowledge about the sequence of the latter. The TCRα repertoires of CD4⁺eGFP⁺ and CD4⁺eGFP⁻ cells from spleens and lymph nodes of naïve mice were similarly analysed. At present, we have a total of 3648 TCRα sequences (from naïve mice: 878 from Treg and 872 from Tconv; from CNS of EAE mice: 981 from Treg and 917 from Tconv).

For analysis, these TCRα sequences were annotated using the IMGT web platform (http://www.imgt.org/), and classified according to repeated occurrences of CDR3 for further analysis. Most sequences were found only once, indicating that the Kaa mouse has a highly polyclonal repertoire, but a few sequences were found a repeated number of times (data not shown). Figure 5 displays the distribution of CDR3 sequences found 8 times or more (among the 3648 sequences) in Treg and Tconv cells from naïve and EAE mice. The two CNS experiments are shown separately, in both cases compared to the entire pool of sequences from Treg and Tconv cells from naïve mice.

The TCRα chains expanded in the CNS Treg compartment showed little overlap with those expanded in CNS Tconv cells, suggesting that these cells have different antigen-recognition properties. The sequences repeated in CNS T cells were barely represented in naïve T cells (Figure 5), suggesting that the corresponding T cells expanded in response to the immunization with MOG, and expressed MOG-reactive TCR. To characterize the antigen-recognition properties associated with the TCR expanded in CNS Treg and Tconv cells, we expressed TCR made of the selected TCRα chains and the Kaa TCRβ chain in the T.54ζ17 T reporter cell line, which is TCR-negative but produces IL-2 after stimulation of the reconstituted TCR by antigen. We selected 16 TCR and made 4 independent cell lines for each of them, which enabled us to show that this assay was reliable to measure the antigen-recognition properties of the TCR because similar IL-2 responses were obtained with the 4 independent cell lines for each of the 16 TCR tested (Figure 6). The data from Figure 6 demonstrate that the Treg-derived TCR conferred T.54ζ17 T cell lines with higher reactivity to MOG than the TCR from Tconv cells, as shown by the higher amounts of IL-2 produced by these lines. In addition, T cell lines expressing Treg-derived TCR responded to lower concentrations of antigen than T cell lines expressing Tconv-derived TCR (not shown).

From these data, we conclude that Treg cells express TCR of higher avidity for MOG than Tconv cells in CNS of Kaa mice. Our data also reveal that the functional avidity of the TCR expressed by Treg cells for MOG is heterogeneous (Figure 6).

### Example 6 - Polyclonal Treg cells engineered to express a MOG-reactive TCR from Kaa mouse fully protect recipient mice from EAE upon adoptive transfer

We then used one of the TCR isolated from MOG-reactive Kaa Treg cells (the one showing the highest functional avidity for MOG) to engineer polyclonal Treg from C57BL/6 mice into MOG-reactive Treg cells. The protective value of these cells was then assessed in adoptive therapy (Figure 7).

Remarkably, re-directed MOG-reactive Treg cells almost completely protected recipient animals from EAE upon adoptive transfer. Thus, it is possible to generate MOG-reactive Treg cells that are strongly protective in EAE by introducing a Kaa mouse-derived TCR into polyclonal Treg cells.

## Claims

1. A transgenic mouse comprising a transgenic expression construct that encodes a MOG-binding T-cell receptor (TCR) β chain under control of a T-cell specific transcriptional promoter,
**characterised in that** the nucleotide sequence that encodes said MOG-binding TCR β chain comprises a sequence that encodes a CDR3 amino acid sequence of SEQ ID No. 1, or an amino acid sequence variant of at least 80% sequence identity, preferably at least 90% sequence identity, to SEQ ID No. 1, whereby the variant exhibits MOG-binding.

2. A transgenic mouse according to the preceding claim, **characterised in that** the T-cell specific transcriptional promoter is a CD2 promoter.

3. A transgenic mouse according to any one of the preceding claims, **characterised in that** the nucleotide sequence that encodes the MOG-binding TCR β chain of claim 1 comprises a nucleotide sequence that encodes an amino acid sequence of SEQ ID No. 2, or an amino acid sequence variant of at least 80% sequence identity, preferably at least 90% sequence identity, to SEQ ID No. 2, whereby the variant exhibits MOG-binding.

4. T-cell receptor (TCR) comprising a MOG-binding TCR β chain of claim 1 and a TCR α chain sequence forming a MOG-binding TCR with the TCR β chain of claim 1.

5. T-cell receptor (TCR) of the preceding claim, comprising a TCR α chain sequence
- with a CDR3 region sequence selected from Table A, or an amino acid sequence variant of at least 80% sequence identity, preferably at least 90% sequence identity, to any one of the CDR3 region sequences of Table A, whereby the variant exhibits MOG-binding, or
- with a VJ region sequence selected from Table A, or an amino acid sequence variant of at least 80% sequence identity, preferably at least 90% sequence identity, to any one of the VJ region sequences of Table A, whereby the variant exhibits MOG-binding.

6. A mouse cell derived or isolated from a mouse according to any one of claims 1 to 3.

7. A cell according to the preceding claim, **characterised in that** the cell is
- a regulatory T-cell (Treg), **characterised by** the markers CD4+, CD25+, and preferably Fox3p+, and optionally additionally **characterised by** expression of markers IL-10, TGF-β and/or IL-35, or
- a pathogenic T-cell (Tconv), **characterised by** the markers CD4+, CD25-, and preferably Fox3p-, and optionally additionally **characterised by** expression of markers IFN-gamma, GM-CSF, IL-17, IL-21, IL-22, TNF, IL-6, lymphotoxin, granzyme, and/or perforin.

8. Method for screening one or more factors for involvement in T-cell therapeutic function, comprising
a. provision of regulatory or pathogenic T-cells defined by the markers according to claim 7 isolated from a mouse,
b. modification of said isolated T-cells via transfection of one or more nucleic acid molecules encoding a MOG-binding TCR, said MOG-binding TCR comprising the MOG-binding TCR β chain of claim 1 and a MOG-binding TCR α chain that forms a MOG-binding TCR with the TCR β chain of claim 1,
c. treatment of modified T-cells with a factor to be investigated, and
d. in vitro assay for T-cell therapeutic function.

9. Method according to claim 8, **characterised in that** the T-cells were isolated directly from the transgenic mouse according to claims 1 to 3 and step b. is not carried out.

10. Method according to claim 8 or 9, **characterised in that** the factor to be investigated is a candidate compound, preferably selected from a small molecule, antibody, anti-sense nucleic acid molecule, cytokine and/or peptide.

11. Method according to any one of claims 8 to 10, whereby step c. is replaced by having used a genetically modified donor mouse in step a., whereby the genetic modification of the donor mouse is the factor to be screened for involvement in T-cell therapeutic function.

12. Method according to any one of claims 8 to 11, whereby the in vitro test of step d. comprises testing for cytokine production in cell culture.

13. Method according to any one of claims 8 to 12, whereby the isolated T-cells of step a. are
- regulatory T-cells (Treg) **characterised by** the markers CD4+, CD25+, and preferably Fox3p+, and is optionally additionally **characterised by** expression of markers IL-10, TGF-β and/or IL-35, or
- pathogenic T-cells (Tconv) **characterised by** the markers CD4+, CD25-, and preferably Fox3p-, and is optionally additionally **characterised by** expression of markers IFN-gamma, GM-CSF, IL-17, IL-21, IL-22, TNF, IL-6, lymphotoxin, granzyme, and/or perforin.

## Patentansprüche

1. Transgene Maus, umfassend ein transgenes Expressionskonstrukt, das für eine MOG-bindende T-Zell-Rezeptor(TCR)-ß-Kette unter Kontrolle eines T-Zell-spezifischen Transkriptionspromoters codiert,
**dadurch gekennzeichnet, dass** die Nukleotidsequenz, welche für die MOG-bindende TCR-ß-Kette codiert, eine Sequenz umfasst, die für eine CDR3-Aminosäuresequenz der SEQ ID Nr. 1 oder eine Aminosäuresequenzvariante von mindestens 80 % Sequenzidentität, vorzugsweise mindestens 90 % Sequenzidentität, mit SEQ ID Nr. 1 codiert, wobei die Variante MOG-Bindung aufweist.

2. Transgene Maus nach dem vorhergehenden Anspruch, **dadurch gekennzeichnet, dass** der T-Zell-spezifische Transkriptionspromotor ein CD2-Promotor ist.

3. Transgene Maus nach einem der vorhergehenden Ansprüche, **dadurch gekennzeichnet, dass** die Nukleotidsequenz, die für die MOG-bindende TCR-ß-Kette nach Anspruch 1 codiert, eine Nukleotidsequenz umfasst, die für eine Aminosäuresequenz der SEQ ID Nr. 2 oder eine Aminosäuresequenzvariante von mindestens 80 % Sequenzidentität, vorzugsweise mindestens 90 % Sequenzidentität, mit SEQ ID Nr. 2 codiert, wobei die Variante MOG-Bindung aufweist.

4. T-Zell-Rezeptor (TCR), umfassend eine MOG-bindende TCR-ß-Kette nach Anspruch 1 und eine TCR-α-Kettensequenz, die einen MOG-bindenden TCR mit der TCR-ß-Kette nach Anspruch 1 bildet.

5. T-Zell-Rezeptor (TCR) nach dem vorhergehenden Anspruch, umfassend eine TCR-α-Kettensequenz
- mit einer aus Tabelle A ausgewählten CDR3-Region-Sequenz oder einer Aminosäuresequenzvariante von mindestens 80 % Sequenzidentität, vorzugsweise mindestens 90 % Sequenzidentität mit einer beliebigen der CDR3-Region-Sequenzen von Tabelle A, wobei die Variante MOG-Bindung aufweist, oder
- mit einer aus Tabelle A ausgewählten VJ-Region-Sequenz oder einer Aminosäuresequenzvariante von mindestens 80 % Sequenzidentität, vorzugsweise mindestens 90 % Sequenzidentität, mit einer beliebigen der VJ-Region-Sequenzen von Tabelle A, wobei die Variante MOG-Bindung aufweist.

6. Mauszelle, die von einer Maus nach einem der Ansprüche 1 bis 3 abgeleitet oder isoliert wird.

7. Zelle nach dem vorhergehenden Anspruch, **dadurch gekennzeichnet, dass** die Zelle
- eine regulatorische T-Zelle (Treg) ist, **gekennzeichnet durch** die Marker CD4+, CD25+ und vorzugsweise Fox3p+ und gegebenenfalls zusätzlich **gekennzeichnet durch** Expression der Marker IL-10, TGF-β und/oder IL-35, oder
- eine pathogene T-Zelle (Tconv) ist, **gekennzeichnet durch** die Marker CD4+, CD25- und vorzugsweise Fox3p- und gegebenenfalls zusätzlich **gekennzeichnet durch** Expression der Marker IFN-gamma, GM-CSF, IL-17, IL-21, IL-22, TNF, IL-6, Lymphotoxin, Granzym und/oder Perforin.

8. Verfahren zum Screenen eines oder mehrerer Faktoren auf Beteiligung an der therapeutischen Funktion von T-Zellen, umfassend
a. Bereitstellung von regulatorischen oder pathogenen T-Zellen, definiert durch die Marker nach Anspruch 7, isoliert von einer Maus,
b. Modifizierung der isolierten T-Zellen durch Transfektion von einer oder mehreren Nukleinsäuremolekülen, die für einen MOG-bindenden TCR codieren, wobei der MOG-bindende TCR die MOG-bindende TCR-ß-Kette nach Anspruch 1 und eine MOG-bindende TCR-α-Kette, die einen MOG-bindenden TCR mit der TCR-ß-Kette nach Anspruch 1 bildet, umfasst,
c. Behandlung von modifizierten T-Zellen mit einem zu untersuchenden Faktor und
d. In-vitro-Assay für die therapeutische Funktion von T-Zellen.

9. Verfahren nach Anspruch 8, **dadurch gekennzeichnet, dass** die T-Zellen direkt aus der transgenen Maus nach den Ansprüchen 1 bis 3 isoliert wurden und Schritt b. nicht ausgeführt wird.

10. Verfahren nach Anspruch 8 oder 9, **dadurch gekennzeichnet, dass** der zu untersuchende Faktor eine Kandidatenverbindung ist, vorzugsweise ausgewählt aus einem kleinen Molekül, einem Antikörper, einem Antisense-Nukleinsäuremolekül, einem Zytokin und/oder einem Peptid.

11. Verfahren nach einem der Ansprüche 8 bis 10, wobei der Schritt c. durch die Verwendung einer genetisch modifizierten Spendermaus in Schritt a. ersetzt wird, wobei die genetische Modifizierung der Spendermaus der Faktor ist, der auf eine Beteiligung an der therapeutischen Funktion von T-Zellen untersucht werden soll.

12. Verfahren nach einem der Ansprüche 8 bis 11, wobei der In-vitro-Test von Schritt d. Testen auf Zytokinproduktion in Zellkultur umfasst.

13. Verfahren nach einem der Ansprüche 8 bis 12, wobei die isolierten T-Zellen von Schritt a.
- regulatorische T-Zellen (Treg) sind, **gekennzeichnet durch** die Marker CD4+, CD25+ und vorzugsweise Fox3p+ und gegebenenfalls zusätzlich **gekennzeichnet durch** Expression der Marker IL-10, TGF-β und/oder IL-35, oder
- pathogene T-Zellen (Tconv) sind, **gekennzeichnet durch** die Marker CD4+, CD25- und vorzugsweise Fox3p- und gegebenenfalls zusätzlich **gekennzeichnet durch** Expression der Marker IFN-gamma, GM-CSF, IL-17, IL-21, IL-22, TNF, IL-6, Lymphotoxin, Granzym und/oder Perforin.

## Revendications

1. Souris transgénique comprenant une construction d'expression transgénique qui code pour une chaîne β du récepteur de lymphocytes T (TCR) se liant à la MOG sous le contrôle d'un promoteur transcriptionnel spécifique aux lymphocytes T,
**caractérisée en ce que** la séquence nucléotidique qui code pour ladite chaîne β du TCR se liant à la MOG comprend une séquence qui code pour une séquence d'acides aminés de CDR3 de la SEQ ID No. 1, ou une séquence d'acides aminés variant d'au moins 80 % d'identité de séquence, de préférence d'au moins 90 % d'identité de séquence par rapport à la SEQ ID No. 1, par laquelle la séquence variante démontre la liaison à la MOG.

2. Souris transgénique selon la revendication précédente, **caractérisée en ce que** le promoteur transcriptionnel spécifique aux lymphocytes T est un promoteur CD2.

3. Souris transgénique selon l'une quelconque des revendications précédentes, **caractérisée en ce que** la séquence nucléotidique qui code pour la chaîne β du TCR se liant à la MOG selon la revendication 1 comprend une séquence nucléotidique qui code pour une séquence d'acides aminés de la SEQ ID No. 2, ou une séquence d'acides aminés variant d'au moins 80 % d'identité de séquence, de préférence d'au moins 90 % d'identité de séquence par rapport à la SEQ ID No. 2, par laquelle la séquence variante démontre la liaison à la MOG.

4. Récepteur de lymphocytes T (TCR) comprenant une chaîne β du TCR se liant à la MOG selon la revendication 1 et une séquence de chaîne α du TCR formant un TCR se liant à la MOG avec la chaîne β TCR selon la revendication 1.

5. Récepteur de lymphocytes T (TCR) selon la revendication précédente, comprenant une séquence de chaîne α du TCR
- avec une séquence de région CDR3 choisie à partir du Tableau A, ou une séquence d'acides aminés variant d'au moins 80 % d'identité de séquence, de préférence d'au moins 90 % d'identité de séquence, par rapport à l'une quelconque des séquences de région CDR3 du Tableau A, par laquelle le variant démontre la liaison à la MOG, ou
- avec une séquence de région VJ choisie à partir du Tableau A, ou une séquence d'acides aminés variant d'au moins 80 % d'identité de séquence, de préférence d'au moins 90 % d'identité de séquence, par rapport à l'une quelconque des séquences de région VJ du Tableau A, par laquelle le variant démontre la liaison à la MOG.

6. Cellule de souris dérivée ou isolée d'une souris selon l'une quelconque des revendications 1 à 3.

7. Cellule selon la revendication précédente, **caractérisée en ce que** la cellule est
- un lymphocyte T régulateur (Treg), **caractérisé par** les marqueurs CD4+, CD25+, et de préférence Fox3p+, et éventuellement également **caractérisé par** l'expression des marqueurs IL-10, TGF-β et/ou IL-35, ou
- un lymphocyte T pathogène (Tconv), **caractérisé par** les marqueurs CD4+, CD25-, et de préférence Fox3p-, et éventuellement également **caractérisé par** l'expression des marqueurs IFN-gamma, GM-CSF, IL-17, IL-21, IL-22, TNF, IL-6, lymphotoxine, granzyme, et/ou perforine.

8. Procédé de criblage d'un ou de plusieurs facteurs pour l'implication dans la fonction thérapeutique des lymphocytes T, comprenant
a. la fourniture de lymphocytes T régulateurs ou pathogènes définis par les marqueurs selon la revendication 7 isolés à partir d'une souris,
b. la modification desdits lymphocytes T isolés par transfection d'une ou de plusieurs molécules d'acide nucléique codant pour un TCR se liant à la MOG, ledit TCR se liant à la MOG comprenant la chaîne β du TCR se liant à la MOG selon la revendication 1 et une chaîne α du TCR se liant à la MOG qui forme un TCR se liant à la MOG avec la chaîne β du TCR selon la revendication 1,
c. le traitement des lymphocytes T modifiés avec un facteur qui doit être étudié, et
d. un test in vitro de la fonction thérapeutique des lymphocytes T.

9. Procédé selon la revendication 8, **caractérisé en ce que** les lymphocytes T ont été isolés directement de la souris transgénique selon les revendications 1 à 3 et l'étape b n'est pas réalisée.

10. Procédé selon la revendication 8 ou 9, **caractérisé en ce que** le facteur qui doit être étudié est un composé candidat, de préférence choisi parmi une petite molécule, un anticorps, une molécule d'acide nucléique anti-sens, une cytokine et/ou un peptide.

11. Procédé selon l'une quelconque des revendications 8 à 10, par lequel l'étape c est remplacée par l'utilisation d'une souris donneuse génétiquement modifiée dans l'étape a, par lequel la modification génétique de la souris donneuse est le facteur qui doit être criblé pour l'implication dans la fonction thérapeutique des lymphocytes T.

12. Procédé selon l'une quelconque des revendications 8 à 11, par lequel le test in vitro de l'étape d comprend l'analyse de la production de cytokine dans la culture cellulaire.

13. Procédé selon l'une quelconque des revendications 8 à 12, par lequel les lymphocytes T isolés de l'étape a sont
- des lymphocytes T régulateurs (Treg), **caractérisés par** les marqueurs CD4+, CD25+, et de préférence Fox3p+, et sont éventuellement également **caractérisés par** l'expression des marqueurs IL-10, TGF-β et/ou IL-35, ou
- des lymphocytes T pathogènes (Tconv), **caractérisés par** les marqueurs CD4+, CD25-, et de préférence Fox3p-, et sont éventuellement également **caractérisés par** l'expression des marqueurs IFN-gamma, GM-CSF, IL-17, IL-21, IL-22, TNF, IL-6, lymphotoxine, granzyme, et/ou perforine.


### SEQUENCE LISTING

<110> Deutsches Rheuma-Forschungszentrum Berlin
<120> TCR transgenic mouse model for immune disease
<130> XII 403/12
<160> 78
<170> PatentIn version 3.3
<210> 1
   <211> 10
   <212> PRT
   <213> Mus musculus
<400> 1
   Gly Glu Thr Gly Asn Asn Tyr Ala Glu Gln
   1               5                   10
<210> 2
   <211> 316
   <212> PRT
   <213> Mus musculus
<400> 2
   Met Ser Asn Thr Ala Phe Pro Asp Pro Ala Trp Asn Thr Thr Leu Leu
   1               5                   10                  15
   Ser Trp Val Ala Leu Phe Leu Leu Gly Thr Lys His Met Glu Ala Ala
               20                  25                  30
   Val Thr Gln Ser Pro Arg Asn Lys Val Ala Val Thr Gly Gly Lys Val
           35                  40                  45
   Thr Leu Ser Cys Asn Gln Thr Asn Asn His Asn Asn Met Tyr Trp Tyr
       50                  55                  60
   Arg Gln Asp Thr Gly His Gly Leu Arg Leu Ile His Tyr Ser Tyr Gly
   65                  70                  75                  80
   Ala Gly Ser Thr Glu Lys Gly Asp Ile Pro Asp Gly Tyr Lys Ala Ser
                   85                  90                  95
   Arg Pro Ser Gln Glu Asn Phe Ser Leu Ile Leu Glu Leu Ala Thr Pro
               100                 105                 110
   Ser Gln Thr Ser Val Tyr Phe Cys Ala Ser Gly Glu Thr Gly Gly Asn
           115                 120                 125
   Tyr Ala Glu Gln Phe Phe Gly Pro Gly Thr Arg Leu Thr Val Leu Glu
       130                 135                 140
   Asp Leu Arg Asn Val Thr Pro Pro Lys Val Ser Leu Phe Glu Pro Ser
   145                 150                 155                 160
   Lys Ala Glu Ile Ala Asn Lys Gln Lys Ala Thr Leu Val Cys Leu Ala
                   165                 170                 175
   Arg Gly Phe Phe Pro Asp His Val Glu Leu Ser Trp Trp Val Asn Gly
               180                 185                 190
   Lys Glu Val His Ser Gly Val Ser Thr Asp Pro Gln Ala Tyr Lys Glu
           195                 200                 205
   Ser Asn Tyr Ser Tyr Cys Leu Ser Ser Arg Leu Arg Val Ser Ala Thr
       210                 215                 220
   Phe Trp His Asn Pro Arg Asn His Phe Arg Cys Gln Val Gln Phe His
   225                 230                 235                 240
   Gly Leu Ser Glu Glu Asp Lys Trp Pro Glu Gly Ser Pro Lys Pro Val
                   245                 250                 255
   Thr Gln Asn Ile Ser Ala Glu Ala Trp Gly Arg Ala Asp Cys Gly Ile
               260                 265                 270
   Thr Ser Ala Ser Tyr His Gln Gly Val Leu Ser Ala Thr Ile Leu Tyr
           275                 280                 285
   Glu Ile Leu Leu Gly Lys Ala Thr Leu Tyr Ala Val Leu Val Ser Gly
       290                 295                 300
   Leu Val Leu Met Ala Met Val Lys Lys Lys Asn Ser
   305                 310                 315
<210> 3
   <211> 13
   <212> PRT
   <213> Mus musculus
<400> 3
   Cys Ala Phe Phe Asn Ser Ala Gly Asn Lys Leu Thr Phe
   1               5                   10
<210> 4
   <211> 109
   <212> PRT
   <213> Mus musculus
<400> 4
   Gly Gln Gln Val Gln Gln Ser Pro Ala Ser Leu Val Leu Gln Glu Gly
   1               5                   10                  15
   Glu Asn Ala Glu Leu Gln Cys Asn Phe Ser Thr Ser Leu Asn Ser Met
               20                  25                  30
   Gln Trp Phe Tyr Gln Arg Pro Gly Gly Ser Leu Val Ser Leu Phe Tyr
           35                  40                  45
   Asn Pro Ser Gly Thr Lys His Ser Gly Arg Leu Thr Ser Thr Thr Val
       50                  55                  60
   Ile Lys Glu Arg Arg Ser Ser Leu His Ile Ser Ser Ser Gln Thr Thr
   65                  70                  75                  80
   Asp Ser Gly Thr Tyr Leu Cys Ala Phe Phe Asn Ser Ala Gly Asn Lys
                   85                  90                  95
   Leu Thr Phe Gly Ile Gly Thr Arg Val Leu Val Arg Pro
               100                 105
<210> 5
   <211> 14
   <212> PRT
   <213> Mus musculus
<400> 5
   Cys Ala Ala Glu Leu Thr Gly Asn Thr Gly Lys Leu Ile Phe
   1               5                   10
<210> 6
   <211> 110
   <212> PRT
   <213> Mus musculus
<400> 6
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Met Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Glu Leu Thr Gly Asn Thr Gly
   85 90 95
   Lys Leu Ile Phe Gly Leu Gly Thr Thr Leu Gln Val Gln Pro
   100 105 110
<210> 7
   <211> 13
   <212> PRT
   <213> Mus musculus
<400> 7
   Cys Ala Ala Glu Ala Gly Ser Gly Thr Tyr Gln Arg Phe
   1 5 10
<210> 8
   <211> 109
   <212> PRT
   <213> Mus musculus
<400> 8
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Ala Gly Ser Gly Thr Tyr
                   85                  90                  95
   Gln Arg Phe Gly Thr Gly Thr Lys Leu Gln Val Val Pro
               100                 105
<210> 9
   <211> 13
   <212> PRT
   <213> Mus musculus
<400> 9
   Cys Ala Ala Glu Ala Gly Ser Gly Thr Tyr Gln Arg Phe
   1               5                   10
<210> 10
   <211> 109
   <212> PRT
   <213> Mus musculus
<400> 10
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Ala Gly Ser Gly Thr Tyr
                   85                  90                  95
   Gln Arg Phe Gly Thr Gly Thr Lys Leu Gln Val Val Pro
               100                 105
<210> 11
   <211> 12
   <212> PRT
   <213> Mus musculus
<400> 11
   Cys Ala Ala Asp Asn Ala Gly Ala Lys Leu Thr Phe
   1               5                   10
<210> 12
   <211> 108
   <212> PRT
   <213> Mus musculus
<400> 12
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Asp Asn Ala Gly Ala Lys Leu
   85 90 95
   Thr Phe Gly Gly Gly Thr Arg Leu Thr Val Arg Pro
   100 105
<210> 13
   <211> 13
   <212> PRT
   <213> Mus musculus
<400> 13
   Cys Ala Ala Arg Asn Thr Asn Thr Gly Lys Leu Thr Phe
   1 5 10
<210> 14
   <211> 109
   <212> PRT
   <213> Mus musculus
<400> 14
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Ile Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Arg Asn Thr Asn Thr Gly Lys
   85 90 95
   Leu Thr Phe Gly Asp Gly Thr Val Leu Thr Val Lys Pro
   100 105
<210> 15
   <211> 13
   <212> PRT
   <213> Mus musculus
<400> 15
   Cys Ala Met Gly Gly Gly Ser Asn Ala Lys Leu Thr Phe
   1               5                   10
<210> 16
   <211> 109
   <212> PRT
   <213> Mus musculus
<400> 16
   Gly Gln Gln Val Gln Gln Ser Pro Thr Ser Leu Val Leu Gln Glu Gly
   1               5                   10                  15
   Glu Asn Ala Glu Leu Gln Cys Asn Phe Ser Thr Ser Leu Asn Ser Met
               20                  25                  30
   Gln Trp Phe Tyr Gln Arg Pro Gly Gly Ser Leu Val Ser Val Phe Tyr
           35                  40                  45
   Asn Pro Ser Gly Thr Lys Gln Ser Gly Arg Leu Thr Ser Thr Thr Val
       50                  55                  60
   Ile Lys Glu Arg Arg Ser Ser Leu His Ile Ser Ser Ser Gln Ile Thr
   65                  70                  75                  80
   Asp Ser Gly Thr Tyr Leu Cys Ala Met Gly Gly Gly Ser Asn Ala Lys
                   85                  90                  95
   Leu Thr Phe Gly Lys Gly Thr Lys Leu Ser Val Lys Ser
               100                 105
<210> 17
   <211> 14
   <212> PRT
   <213> Mus musculus
<400> 17
   Cys Ala Ala Thr Ala Ser Ser Ser Phe Ser Lys Leu Val Phe
   1               5                   10
<210> 18
   <211> 110
   <212> PRT
   <213> Mus musculus
<400> 18
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Thr Ala Ser Ser Ser Phe Ser
                   85                  90                  95
   Lys Leu Val Phe Gly Gln Gly Thr Ser Leu Ser Val Val Pro
               100                 105                 110
<210> 19
   <211> 11
   <212> PRT
   <213> Mus musculus
<400> 19
   Cys Ala Ala Gly Asn Tyr Asn Val Leu Tyr Phe
   1               5                   10
<210> 20
   <211> 107
   <212> PRT
   <213> Mus musculus
<400> 20
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gly Asn Tyr Asn Val Leu Tyr
                   85                  90                  95
   Phe Gly Ser Gly Thr Lys Leu Thr Val Glu Pro
               100                 105
<210> 21
   <211> 13
   <212> PRT
   <213> Mus musculus
<400> 21
   Cys Ala Ala Asp Ser Ser Gly Ser Trp Gln Leu Ile Phe
   1               5                   10
<210> 22
   <211> 109
   <212> PRT
   <213> Mus musculus
<400> 22
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Asp Ser Ser Gly Ser Trp Gln
                   85                  90                  95
   Leu Ile Phe Gly Ser Gly Thr Gln Leu Thr Val Met Pro
               100                 105
<210> 23
   <211> 11
   <212> PRT
   <213> Mus musculus
<400> 23
   Cys Ala Ala Gly Asn Tyr Asn Val Leu Tyr Phe
   1               5                   10
<210> 24
   <211> 107
   <212> PRT
   <213> Mus musculus
<400> 24
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gly Asn Tyr Asn Val Leu Tyr
                   85                  90                  95
   Phe Gly Ser Gly Thr Lys Leu Thr Val Glu Pro
               100                 105
<210> 25
   <211> 13
   <212> PRT
   <213> Mus musculus
<400> 25
   Cys Ala Ala Arg Asn Thr Asn Thr Gly Lys Leu Thr Phe
   1               5                   10
<210> 26
   <211> 109
   <212> PRT
   <213> Mus musculus
<400> 26
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Arg Asn Thr Asn Thr Gly Lys
   85 90 95
   Leu Thr Phe Gly Asp Gly Thr Val Leu Thr Val Lys Pro
   100 105
<210> 27
   <211> 13
   <212> PRT
   <213> Mus musculus
<400> 27
   Cys Ala Pro Thr Asn Ser Ala Gly Asn Lys Leu Thr Phe
   1 5 10
<210> 28
   <211> 109
   <212> PRT
   <213> Mus musculus
<400> 28
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Ile Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Pro Thr Asn Ser Ala Gly Asn Lys
   85 90 95
   Leu Thr Phe Gly Ile Gly Thr Arg Val Leu Val Arg Pro
   100 105
<210> 29
   <211> 11
   <212> PRT
   <213> Mus musculus
<400> 29
   Cys Ala Ala Asp Tyr Ala Asn Lys Met Ile Phe
   1               5                   10
<210> 30
   <211> 107
   <212> PRT
   <213> Mus musculus
<400> 30
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Asp Tyr Ala Asn Lys Met Ile
                   85                  90                  95
   Phe Gly Leu Gly Thr Ile Leu Arg Val Arg Pro
               100                 105
<210> 31
   <211> 13
   <212> PRT
   <213> Mus musculus
<400> 31
   Cys Ala Ala Ser Lys Ala Asn Thr Gly Lys Leu Thr Phe
   1               5                   10
<210> 32
   <211> 111
   <212> PRT
   <213> Mus musculus
<400> 32
   Gln Gln Lys Val Gln Gln Ser Pro Glu Ser Leu Ile Val Pro Glu Gly
   1               5                   10                  15
   Gly Met Ala Ser Leu Asn Cys Thr Ser Ser Asp Arg Asn Val Asp Tyr
               20                  25                  30
   Phe Trp Trp Tyr Arg Gln His Ser Gly Lys Ser Pro Lys Met Leu Met
           35                  40                  45
   Ser Ile Phe Ser Asn Gly Glu Lys Glu Glu Gly Arg Phe Thr Val His
       50                  55                  60
   Leu Asn Lys Ala Ser Leu His Thr Ser Leu His Ile Arg Asp Ser Gln
   65                  70                  75                  80
   Pro Ser Asp Ser Ala Leu Tyr Leu Cys Ala Ala Ser Lys Ala Asn Thr
                   85                  90                  95
   Gly Lys Leu Thr Phe Gly Asp Gly Thr Val Leu Thr Val Lys Pro
               100                 105                 110
<210> 33
   <211> 12
   <212> PRT
   <213> Mus musculus
<400> 33
   Cys Ala Ala Ala Asn Tyr Ala Gln Gly Leu Thr Phe
   1               5                   10
<210> 34
   <211> 108
   <212> PRT
   <213> Mus musculus
<400> 34
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Ala Asn Tyr Ala Gln Gly Leu
                   85                  90                  95
   Thr Phe Gly Leu Gly Thr Arg Val Ser Val Phe Pro
               100                 105
<210> 35
   <211> 12
   <212> PRT
   <213> Mus musculus
<400> 35
   Cys Ala Ala Pro Gly Asn Thr Gly Lys Leu Ile Phe
   1               5                   10
<210> 36
   <211> 108
   <212> PRT
   <213> Mus musculus
<400> 36
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Pro Gly Asn Thr Gly Lys Leu
                   85                  90                  95
   Ile Phe Gly Leu Gly Thr Thr Leu Gln Val Gln Pro
               100                 105
<210> 37
   <211> 11
   <212> PRT
   <213> Mus musculus
<400> 37
   Cys Ala Ala Gln Gly Gly Arg Ala Leu Ile Phe
   1               5                   10
<210> 38
   <211> 107
   <212> PRT
   <213> Mus musculus
<400> 38
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gln Gly Gly Arg Ala Leu Ile
                   85                  90                  95
   Phe Gly Thr Gly Thr Thr Val Ser Val Ser Pro
               100                 105
<210> 39
   <211> 12
   <212> PRT
   <213> Mus musculus
<400> 39
   Cys Ala Ala Thr Ser Ser Gly Gln Lys Leu Val Phe
   1               5                   10
<210> 40
   <211> 108
   <212> PRT
   <213> Mus musculus
<400> 40
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Ile Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Thr Ser Ser Gly Gln Lys Leu
   85 90 95
   Val Phe Gly Gln Gly Thr Ile Leu Lys Val Tyr Leu
   100 105
<210> 41
   <211> 12
   <212> PRT
   <213> Mus musculus
<400> 41
   Cys Ala Ala Glu Ala Gly Asn Asn Arg Ile Phe Phe
   1 5 10
<210> 42
   <211> 108
   <212> PRT
   <213> Mus musculus
<400> 42
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Ala Gly Asn Asn Arg Ile
                   85                  90                  95
   Phe Phe Gly Asp Gly Thr Gln Leu Val Val Lys Pro
               100                 105
<210> 43
   <211> 11
   <212> PRT
   <213> Mus musculus
<400> 43
   Cys Ala Ala Gln Gly Gly Arg Ala Leu Ile Phe
   1               5                   10
<210> 44
   <211> 107
   <212> PRT
   <213> Mus musculus
<400> 44
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gln Gly Gly Arg Ala Leu Ile
                   85                  90                  95
   Phe Gly Thr Gly Thr Thr Val Ser Val Ser Pro
               100                 105
<210> 45
   <211> 10
   <212> PRT
   <213> Mus musculus
<400> 45
   Cys Ala Ala Ser Asn Tyr Gln Leu Ile Trp
   1               5                   10
<210> 46
   <211> 106
   <212> PRT
   <213> Mus musculus
<400> 46
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Ser Asn Tyr Gln Leu Ile Trp
   85 90 95
   Gly Ser Gly Thr Lys Leu Ile Ile Lys Pro
   100 105
<210> 47
   <211> 13
   <212> PRT
   <213> Mus musculus
<400> 47
   Cys Ala Ala His Asn Thr Asn Thr Gly Lys Leu Thr Phe
   1 5 10
<210> 48
   <211> 109
   <212> PRT
   <213> Mus musculus
<400> 48
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Met Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala His Asn Thr Asn Thr Gly Lys
   85 90 95
   Leu Thr Phe Gly Asp Gly Thr Val Leu Thr Val Lys Pro
   100 105
<210> 49
   <211> 13
   <212> PRT
   <213> Mus musculus
<400> 49
   Cys Ala Ala Gly Asn Ser Ala Gly Asn Lys Leu Thr Phe
   1               5                   10
<210> 50
   <211> 109
   <212> PRT
   <213> Mus musculus
<400> 50
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gly Asn Ser Ala Gly Asn Lys
                   85                  90                  95
   Leu Thr Phe Gly Ile Gly Thr Arg Val Leu Val Arg Pro
               100                 105
<210> 51
   <211> 14
   <212> PRT
   <213> Mus musculus
<400> 51
   Cys Ala Ala Glu Val Ala Gly Asn Thr Gly Lys Leu Ile Phe
   1               5                   10
<210> 52
   <211> 110
   <212> PRT
   <213> Mus musculus
<400> 52
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Val Ala Gly Asn Thr Gly
                   85                  90                  95
   Lys Leu Ile Phe Gly Leu Gly Thr Thr Leu Gln Val Gln Pro
               100                 105                 110
<210> 53
   <211> 14
   <212> PRT
   <213> Mus musculus
<400> 53
   Cys Ala Ala Glu Pro Ala Arg Ser Asn Ala Lys Leu Thr Phe
   1               5                   10
<210> 54
   <211> 110
   <212> PRT
   <213> Mus musculus
<400> 54
   Gly Asp Gln Val Lys Gln Ser Pro Ser Ala Leu Ser Leu Gln Glu Gly
   1               5                   10                  15
   Thr Asn Ser Ala Leu Arg Cys Asn Phe Ser Ile Ala Thr Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Thr Phe Asn
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Pro Ala Arg Ser Asn Ala
                   85                  90                  95
   Lys Leu Thr Phe Gly Lys Gly Thr Lys Leu Ser Val Lys Ser
               100                 105                 110
<210> 55
   <211> 14
   <212> PRT
   <213> Mus musculus
<400> 55
   Cys Ala Ala Glu Leu Thr Gly Asn Thr Gly Lys Leu Ile Phe
   1               5                   10
<210> 56
   <211> 110
   <212> PRT
   <213> Mus musculus
<400> 56
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Leu Thr Gly Asn Thr Gly
                   85                  90                  95
   Lys Leu Ile Phe Gly Leu Gly Thr Thr Leu Gln Val Gln Pro
               100                 105                 110
<210> 57
   <211> 11
   <212> PRT
   <213> Mus musculus
<400> 57
   Cys Ala Ala Ser Asn Thr Asn Lys Val Val Phe
   1               5                   10
<210> 58
   <211> 107
   <212> PRT
   <213> Mus musculus
<400> 58
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Ser Asn Thr Asn Lys Val Val
                   85                  90                  95
   Phe Gly Thr Gly Thr Arg Leu Gln Val Leu Pro
               100                 105
<210> 59
   <211> 14
   <212> PRT
   <213> Mus musculus
<400> 59
   Cys Ala Ala Glu Pro Ala Arg Ser Asn Ala Lys Leu Thr Phe
   1               5                   10
<210> 60
   <211> 110
   <212> PRT
   <213> Mus musculus
<400> 60
   Gly Asp Gln Val Lys Gln Ser Pro Ser Ala Leu Ser Leu Gln Glu Gly
   1               5                   10                  15
   Thr Asn Ser Ala Leu Arg Cys Asn Phe Ser Ile Ala Thr Thr Thr Val
               20                  25                  30
   Gln Trp Phe Leu Gln Asn Pro Arg Gly Ser Leu Met Asn Leu Phe Tyr
           35                  40                  45
   Leu Val Pro Gly Thr Lys Glu Asn Gly Arg Leu Lys Ser Thr Phe Asn
       50                  55                  60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Glu Pro Ala Arg Ser Asn Ala
   85 90 95
   Lys Leu Thr Phe Gly Lys Gly Thr Lys Leu Ser Val Lys Ser
   100 105 110
<210> 61
   <211> 13
   <212> PRT
   <213> Mus musculus
<400> 61
   Cys Ala Ala Ser Gly Ala Asn Thr Gly Lys Leu Thr Phe
   1 5 10
<210> 62
   <211> 111
   <212> PRT
   <213> Mus musculus
<400> 62
   Gln Gln Lys Val Gln Gln Ser Pro Glu Ser Leu Ile Val Pro Glu Gly
   1 5 10 15
   Gly Met Ala Ser Leu Asn Cys Thr Ser Ser Asp Arg Asn Val Asp Tyr
   20 25 30
   Phe Trp Trp Tyr Arg Gln His Ser Gly Lys Ser Pro Lys Met Leu Met
   35 40 45
   Ser Ile Phe Ser Asn Gly Glu Lys Glu Glu Gly Arg Phe Thr Val His
   50 55 60
   Leu Asn Lys Ala Ser Leu His Thr Ser Leu His Ile Arg Asp Ser Gln
   65 70 75 80
   Pro Ser Asp Ser Ala Leu Tyr Leu Cys Ala Ala Ser Gly Ala Asn Thr
   85 90 95
   Gly Lys Leu Thr Phe Gly Asp Gly Thr Val Leu Thr Val Lys Pro
   100 105 110
<210> 63
   <211> 12
   <212> PRT
   <213> Mus musculus
<400> 63
   Cys Ala Ala Gly Asn Ser Asn Asn Arg Ile Phe Phe
   1               5                   10
<210> 64
   <211> 108
   <212> PRT
   <213> Mus musculus
<400> 64
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Gly Asn Ser Asn Asn Arg Ile
                   85                  90                  95
   Phe Phe Gly Asp Gly Thr Gln Leu Val Val Lys Pro
               100                 105
<210> 65
   <211> 13
   <212> PRT
   <213> Mus musculus
<400> 65
   Cys Ala Ala Ser Gly Ala Asn Thr Gly Lys Leu Thr Phe
   1               5                   10
<210> 66
   <211> 111
   <212> PRT
   <213> Mus musculus
<400> 66
   Gln Gln Lys Val Gln Gln Ser Pro Glu Ser Leu Ile Val Pro Glu Gly
   1               5                   10                  15
   Gly Met Ala Ser Leu Asn Cys Thr Ser Ser Asp Arg Asn Val Asp Tyr
               20                  25                  30
   Phe Trp Trp Tyr Arg Gln His Ser Gly Lys Ser Pro Lys Met Leu Met
           35                  40                  45
   Ser Ile Phe Ser Asn Gly Glu Lys Glu Glu Gly Arg Phe Thr Val His
       50                  55                  60
   Leu Asn Lys Ala Ser Leu His Thr Ser Leu His Ile Arg Asp Ser Gln
   65                  70                  75                  80
   Pro Ser Asp Ser Ala Leu Tyr Leu Cys Ala Ala Ser Gly Ala Asn Thr
                   85                  90                  95
   Gly Lys Leu Thr Phe Gly His Gly Thr Ile Leu Arg Val His Pro
               100                 105                 110
<210> 67
   <211> 14
   <212> PRT
   <213> Mus musculus
<400> 67
   Cys Ala Ala Glu Ile Thr Gly Asn Thr Gly Lys Leu Ile Phe
   1               5                   10
<210> 68
   <211> 110
   <212> PRT
   <213> Mus musculus
<400> 68
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Glu Ile Thr Gly Asn Thr Gly
                   85                  90                  95
   Lys Leu Ile Phe Gly Leu Gly Thr Thr Leu Gln Val Gln Pro
               100                 105                 110
<210> 69
   <211> 13
   <212> PRT
   <213> Mus musculus
<400> 69
   Cys Ala Ala Pro Val Thr Asn Ala Tyr Lys Val Ile Phe
   1               5                   10
<210> 70
   <211> 109
   <212> PRT
   <213> Mus musculus
<400> 70
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Pro Val Thr Asn Ala Tyr Lys
                   85                  90                  95
   Val Ile Phe Gly Lys Gly Thr His Leu His Val Leu Pro
               100                 105
<210> 71
   <211> 13
   <212> PRT
   <213> Mus musculus
<400> 71
   Cys Ala Ala Asp Gly Gly Ser Asn Ala Lys Leu Thr Phe
   1               5                   10
<210> 72
   <211> 109
   <212> PRT
   <213> Mus musculus
<400> 72
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Ile Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Asp Gly Gly Ser Asn Ala Lys
   85 90 95
   Leu Thr Phe Gly Lys Gly Thr Lys Leu Ser Val Lys Ser
   100 105
<210> 73
   <211> 13
   <212> PRT
   <213> Mus musculus
<400> 73
   Cys Ala Ala Pro Val Thr Asn Ala Tyr Lys Val Ile Phe
   1 5 10
<210> 74
   <211> 109
   <212> PRT
   <213> Mus musculus
<220>
   <221> misc_feature
   <222> (58)..(58)
   <223> Xaa can be any naturally occurring amino acid
<400> 74
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Met Asn Leu Phe Tyr
   35 40 45
   Leu Val Pro Gly Thr Lys Glu Asn Gly Xaa Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Pro Val Thr Asn Ala Tyr Lys
   85 90 95
   Val Ile Phe Gly Lys Gly Thr His Leu His Val Leu Pro
   100 105
<210> 75
   <211> 11
   <212> PRT
   <213> Mus musculus
<400> 75
   Cys Ala Ala Ala Ser Asn Asn Arg Ile Phe Phe
   1 5 10
<210> 76
   <211> 107
   <212> PRT
   <213> Mus musculus
<400> 76
   Gly Asp Lys Val Lys Gln Ser Pro Ser Ala Leu Ser Leu Gln Glu Gly
   1 5 10 15
   Thr Asn Ser Ala Leu Arg Cys Asn Phe Ser Ile Ala Ala Thr Thr Val
   20 25 30
   Gln Trp Phe Leu Gln Asn Pro Arg Gly Ser Leu Ile Asn Leu Phe Tyr
   35 40 45
   Leu Val Pro Gly Thr Lys Glu Asn Gly Arg Leu Lys Ser Ala Phe Asp
   50 55 60
   Ser Lys Glu Ser Tyr Ser Thr Leu His Ile Arg Asp Ala Gln Leu Glu
   65 70 75 80
   Asp Ser Gly Thr Tyr Phe Cys Ala Ala Ala Ser Asn Asn Arg Ile Phe
   85 90 95
   Phe Gly Asp Gly Thr Gln Leu Val Val Lys Pro
   100 105
<210> 77
   <211> 11
   <212> PRT
   <213> Mus musculus
<400> 77
   Cys Ala Ala Asn Tyr Ala Gln Gly Leu Thr Phe
   1               5                   10
<210> 78
   <211> 107
   <212> PRT
   <213> Mus musculus
<400> 78
   Gly Asp Lys Val Lys Gln Ser Pro Ser Ala Leu Ser Leu Gln Glu Gly
   1               5                   10                  15
   Thr Asn Ser Ala Leu Arg Cys Asn Phe Ser Ile Ala Ala Thr Thr Val
               20                  25                  30
   Gln Trp Phe Leu Gln Asn Pro Arg Gly Ser Leu Ile Asn Leu Phe Tyr
           35                  40                  45
   Leu Val Pro Gly Thr Lys Glu Asn Gly Arg Leu Lys Ser Ala Phe Asp
       50                  55                  60
   Ser Lys Glu Ser Tyr Ser Thr Leu His Ile Arg Asp Ala Gln Leu Glu
   65                  70                  75                  80
   Asp Ser Gly Thr Tyr Phe Cys Ala Ala Asn Tyr Ala Gln Gly Leu Thr
                   85                  90                  95
   Phe Gly Leu Gly Thr Arg Val Ser Val Phe Pro
               100                 105